# EUROPÄISCHE PATENTSCHRIFT

(11) **EP 2 308 539 B1**
(45) Veröffentlichungstag und Bekanntmachung des Hinweises auf die Patenterteilung: **20.04.2016**
(21) Anmeldenummer: 10185455.2
(22) Anmeldetag: 04.08.2000
(51) Int. Cl.: A61M 16/08, A61M 16/16, A61M 16/10, A62B 9/00

(54) **Vorrichtung zur Zufuhr eines Atemgases, Befeuchtungsvorrichtung, Atemgasschlauch und Anschlussvorrichtung hierfür**
Device for supplying respiratory gas, humidifying device, respiratory gas tube, and connecting device therefor
Dispositif pour délivrer un gaz respiratoire, humidificateur, tube respiratoire, et dispositif de connection

(30) Priorität: 05.08.1999 DE 19936499; 13.10.1999 DE 19949292; 13.10.1999 DE 19949283; 13.10.1999 DE 29918048 U
(43) Veröffentlichungstag der Anmeldung: 13.04.2011
(62) Teilanmeldung aus: 06006804.6
(73) Patentinhaber: ResMed R&D Germany GmbH, 82152 Martinsried (DE)
(72) Erfinder: Mayer, Wolfgang, 79285, Ebringen (DE); Genger, Harald, 82319, Starnberg (DE); Madaus, Stefan, 82166 Gräfelfing (DE); Klopp, Andreas, 81243, München (DE); Schätzl, Stefan, 82362, Weilheim (DE); Vögele, Harald, 82131, Gauting (DE); Lang, Bernd, 82166, Gräfelfing (DE)
(74) Vertreter: Vossius & Partner Patentanwälte Rechtsanwälte mbB

(56) Entgegenhaltungen:
- EP-A- 0 903 160
- WO-A-97/32619
- WO-A1-98/57691
- WO-A1-99/22793
- DE-A1- 19 630 466
- DE-U1- 29 817 685
- US-A- 5 564 415

## Beschreibung

Die Erfindung betrifft eine Vorrichtung zur Zufuhr eines Atemgases unter Überdruck und eine Befeuchtungsvorrichtung für eine Vorrichtung zur Zufuhr eines Atemgases unter Überdruck.

Eine Vorrichtung zur Zufuhr eines Atemgases unter Überdruck mit einer Gebläseeinrichtung zur Förderung des Atemgases, einer Gehäuseeinrichtung zur Aufnahme der Gebläseeinrichtung und einer Anschlußeinrichtung zum Anschluß einer Befeuchtungsvorrichtung zur Befeuchtung des seitens der Fördereinrichtung geförderten Atemgases wird diskutiert. Ferner werden eine Befeuchtungsvorrichtung zur Befeuchtung eines Atemgases sowie einen Atemgasschlauch und eine Anschlußvorrichtung hierfür diskutiert.

WO 99/22793 A1, WO 98/57691 A1 und US 5,564,415 offenbaren jeweils eine Vorrichtung zur Zufuhr von Gas zu einer Person sowie einen Befeuchter.

Vorrichtungen zur Zufuhr eines Atemgases unter Überdruck finden insbesondere im Bereich der Schlaftherapie zur Behandlung schlafbezogener Atmungsstörungen Anwendung. Durch Zuführung des Atemgases unter einem vorbestimmten Überdruck üblicherweise im Bereich von 5-20 mbar wird auf physiologisch gut verträgliche Weise eine pneumatische Schienung der oberen Atemwege eines Patienten erreicht, wodurch einer Obstruktion dieses Atemwegsbereiches auf wirkungsvolle Weise vorgebeugt werden kann.

Üblicherweise wird das Atemgas unmittelbar aus der vorzugsweise über eine Filtereinrichtung angesaugten Umgebungsluft gebildet. In Abhängigkeit von den insbesondere jahreszeitlich bedingt schwankenden klimatischen Verhältnissen hat es sich als vorteilhaft erwiesen, das dem Patienten beispielsweise über eine Gebläseeinrichtung unter einem geregelten, ggf. alternierenden Druck, zugeführte Atemgas zeitweise zu befeuchten. Hierzu ist es möglich, beispielsweise über ein Schlauchzwischenstück eine Befeuchtungseinrichtung in den Atemgasweg zwischen Gebläseeinrichtung und Atemmaske einzufügen. Es sind auch CPAP-Geräte mit integrierter Befeuchtungsvorrichtung bekannt.

Bei den lediglich in eine Schlauchleitung eingesteckten Befeuchtungsvorrichtungen besteht jedoch häufig das Problem einer ungenügenden Standfestigkeit. Bei CPAP-Geräten mit integrierter Befeuchtungsvorrichtung muß diese ständig mitgeführt werden, auch wenn vorübergehend kein Bedarf nach einer Befeuchtung des Atemgases besteht.

Unter dem Eindruck dieses Problems liegt der Erfindung die Aufgabe zugrunde, eine Vorrichtung zur Zufuhr eines Atemgases sowie eine hierfür vorgesehene Befeuchtungsvorrichtung zu schaffen, die robust und einfach handhabbar sowie auf einfache Weise bedarfsgerecht konfigurierbar ist.

Die Erfindung ist durch die Ansprüche definiert.

Durch die hierin diskutierten Vorrichtungen wird es auf vorteilhafte Weise möglich, einfach und ohne Bedarf nach einer fachmännischen Montagetechnik eine Befeuchtungsvorrichtung unmittelbar seitlich an ein CPAP-Gerät anzukoppeln, ohne daß das CPAP-Gerät hierzu angehoben werden muß. In vorteilhafter Weise wirken hierbei untere Aufstellabschnitte über welche das CPAP-Gerät aufgestellt ist, unmittelbar als Führungseinrichtung, die ein einfaches Anschieben der Befeuchtungsvorrichtung an das CPAP-Gerät ermöglicht. Besteht beispielsweise vorübergehend kein Bedarf nach einer Befeuchtungsvorrichtung, oder soll die Befeuchtungsvorrichtung zum Zwecke der Reinigung vorübergehend von dem CPAP-Gerät getrennt werden, so kann das CPAP-Gerät unverändert an seinem Aufstellungsort verbleiben, und die Befeuchtungsvorrichtung kann einfach zur Seite hin abgenommen insbesondere abgezogen werden.

Gemäß einer besonders bevorzugten Ausführungsform der Erfindung sind die Anschlußorgane im wesentlichen in Fügerichtung ausgerichtet. Insbesondere der Hauptdurchgangsquerschnitt für das seitens der Gebläseeinrichtung geförderte Atemgas ist in vorteilhafter Weise durch einen Rohrstutzen gebildet, auf welchen ein seitens einer entsprechend komplementär ausgebildeten Befeuchtungsvorrichtung vorgesehener Anschlußabschnitt aufgesteckt werden kann.

Eine insbesondere unter ästhetischen Gesichtspunkten sowie im Hinblick auf einen symmetrischen Aufbau des CPAP-Gerätesystems vorteilhafte Ausführungsform der Erfindung ist dadurch gegeben, daß die entsprechenden Anschlußorgane in einer Geräte-Stirnseite (Frontseite) ausgebildet sind. Der Flächenabschnitt dieser Geräte-Stirnseite ist im wesentlichen komplementär zu einem in Fügestellung benachbarten Abschnitt der Befeuchtungsvorrichtung ausgebildet.

Eine insbesondere im Hinblick' auf eine besonders zuverlässige Ankoppelung einer Druckmeßleitung vorteilhafte Ausführungsform der Erfindung ist dadurch gegeben, daß die Anschlußeinrichtung den besagten Rohrstutzen zur Durchleitung des Atemgases und einen diesem benachbart angeordneten Leitungsabschnitt zur Ankoppelung einer Druckmeßleitung aufweist.

Der Rohrstutzen für die Durchleitung des Atemgases und der Leitungsabschnitt für die Druckmeßleitung sind gemäß einer besonders bevorzugten Ausführungsform der Erfindung in einer Ausnehmung derart angeordnet, daß diese im wesentlichen nicht über eine durch die vordere Stirnfläche des Gerätes definierte Hauptebene überstehen. Hierdurch ist ein besonders wirkungsvoller Schutz dieser vergleichsweise filigranen CPAP-Gerät-Anschlußorgane gegeben.

Gemäß einem besonderen Aspekt der vorliegenden Erfindung umfaßt die Anschlußeinrichtung zum bedarfsweisen Anschluß der Befeuchtungseinrichtung-Elektroanschlußorgane zur Schaffung einer elektrischen Verbindung mit der Befeuchtungsvorrichtung. Über diese Elektroanschlußorgane wird es auf vorteilhafte Weise möglich, eine Heizeinrichtung der Befeuchtungsvorrichtung mit Spannung zu versorgen, ohne, daß hierzu manuell ein entsprechendes Spannungsversorgungskabel an die Befeuchtungsvorrichtung angeschlossen werden muß. Die Elektroanschlußorgane können auch zur Übertragung elektrischer Signale, beispielsweise zur Übertragung eines Füllstandssignales oder auch zur Übertragung elektrischer Signale verwendet werden, die beispielsweise im Bereich der Atemschlauchanschlußeinrichtung zugeführt wurden.

Eine besonders wirkungsvolle Koppelung des CPAP-Gerätes mit der zum Anschluß hieran vorgesehenen Befeuchtungsvorrichtung wird erfindungsgemäß dadurch erreicht, daß eine manuell in Lösestellung bringbare Verrastungseinrichtung vorgesehen ist, die die Befeuchtungsvorrichtung in einer Fügestellung hält. Dadurch wird es auf vorteilhafte Weise möglich, die Befeuchtungsvorrichtung äußerst gewichtssparend auszubilden, ohne daß hierbei die Gefahr besteht, daß diese versehentlich über den angeschlossenen Atemgasschlauch vom CPAP-Gerät abgezogen und von ihrer Aufstellfläche (z.B. Beistelltisch) heruntergezogen wird.

Das CPAP-Gerät ist gem. einer besonders bevorzugten Ausführungsform der Erfindung im Bodenbereich derart ausgebildet, daß die Anschlußorgane, insbesondere der genannte Rohrstutzen auf einem vertikalen Höhenniveau angeordnet sind, das exakt dem Höhenniveau der seitens der Befeuchtungsvorrichtung vorgesehenen Anschlußorgane entspricht.

In vorteilhafter Weise sind die seitens des CPAP-Gerätes als auch die seitens der Befeuchtungsvorrichtung vorgesehenen Anschlußorgane in vertikaler Richtung derart positioniert, daß bei Aufstellung des CPAP-Gerätes und der Befeuchtungsvorrichtung auf einer im wesentlichen planen Unterlage die Befeuchtungsvorrichtung an das CPAP-Gerät herangeschoben werden kann, wobei die erforderliche Ausrichtung dieser beiden Module in vertikaler Richtung bereits durch die Standfläche erreicht wird. Um auch in seitlicher Richtung eine ausreichende Zentrierung der beiden Module zu erreichen, sind gem. einer besonders bevorzugten Ausführungsform der Erfindung ebenfalls Zentrierhilfen vorgesehen. Eine besonders robuste Zentrierhilfe wird hierbei erreicht, indem die Innenwandung der den Rohrstutzen ausnehmenden Ausnehmung auf die Außenumfangsfläche des seitens der Befeuchtungsvorrichtung vorgesehenen Anschlußzapfens abgestimmt ist.

Hinsichtlich einer Befeuchtungsvorrichtung wird die eingangs angegebene Aufgabe durch eine hierin diskutierte Befeuchtungsvorrichtung gelöst. Eine derartige Befeuchtungsvorrichtung kann auf einfache Weise auch von einem Laien an ein entsprechendes CPAP-Gerät angekoppelt werden, ohne daß es hierzu einer fachmännischen Montagetechnik oder eines Verbindungsschlauches bedarf. Das CPAP-Gerät muß hierzu nicht angehoben werden.

In vorteilhafter Weise wird eine an ein Basisgerät ankoppelbare Befeuchtereinheit geschaffen welche ein kartuschenartiges entnehmbares und wieder einsetzbares Nachfüllmodul umfaßt. Das Nachfüllmodul kann über Fixiereinrichtungen beispielsweise eine Bajonetverschlußeinrichtung in der Befeuchtereinheit fixiert werden. Durch Dichteinrichtungen kann das Nachfüllmodul abschnittsweise oder vollständig in der Befeuchtereinheit abgedichtet werden.

Im Rahmen einer CPAP-Therapie erfolgt eine Unterstützung der Spontanatmung eines Patienten, indem diesem ein Atemgas unter permantentem Überdruck zugeführt wird. Durch diesen Überdruck wird eine pneumatische Schienung der oberen Atemwege erreicht, wodurch etwaigen während der Schlafphase eines Patienten auftretenden Atemwegsobstruktionen vorgebeugt werden kann. Bei einer derartigen Behandlung schlafbezogener Atmungsstörungen erstreckt sich diese Überdruckbeatmung üblicherweise über die gesamte Schlafphase des Patienten. Im Hinblick auf eine verbesserte physiologische Verträglichkeit der Überdruckbeatmung hat es sich als vorteilhaft erwiesen, das dem Patienten zugeführte Atemgas zu befeuchten. Üblicherweise erfolgt die Befeuchtung des Atemgases, indem dieses über ein Wasserbad geführt wird und hierbei Feuchtigkeit aufnimmt. In diesem Wasserbad wird üblicherweise eine Wassermenge von ca. 750ml bevorratet. Das Wasserbad wird vorzugsweise mittels einer Heizeinrichtung leicht erwärmt. Bei diesen herkömmlichen Befeuchtungsvorrichtungen hat sich gezeigt, daß die absolute Feuchtigkeit des Atemgases über die gesamte Schlafphase gesehen teilweise erheblichen Schwankungen unterliegt.

Zur Lösung dieses Problems wird gemäß einem besonderen Asparkt der vorliegenden Erfindung, eine einfach handhabbare Vorrichtung zur Befeuchtung eines Atemgases sowie ein zur Verwendung hiermit vorgesehenes CPAP-Gerät beschrieben, durch welche, bzw. durch welches, eine gleichmäßige Befeuchtung des Atemgases erreicht werden kann. Dies wird erreicht durch eine Vorrichtung zur Befeuchtung eines Atemgases mit einem Flüssigkeitsvorratsraum zur Bevorratung einer Flüssigkeit, einem Befeuchtungsbereich zur Befrachtung des Atemgases mit der Flüssigkeit, indem das Atemgas in dem Befeuchtungsbereich mit der Flüssigkeit in Kontakt tritt, einer Atemgaszuleitungseinrichtung zur Zuleitung des Atemgases zu dem Befeuchtungsbereich, und einer Atemgasableitungseinrichtung zur Ableitung des befeuchteten Atemgases aus dem Befeuchtungsbereich, wobei eine Teilmengenabgabeeinrichtung vorgesehen ist, zur Weitergabe lediglich einer Teilmenge der in dem Flüssigkeitsvorratsraum bevorrateten Flüssigkeit in den Befeuchtungsbereich.

Dadurch wird es auf vorteilhafte Weise möglich, bereits kurzfristig nach Inbetriebnahme des Gerätes ein bedarfsgerecht befeuchtetes Atemgas bereitzustellen. Bei gewünschter Erwärmung des Befeuchtungsmediums kann diese rasch und unter vergleichsweise geringem Leistungsbezug erreicht werden. Infolge des geringen Leistungsbezugs der Heizeinrichtung eignet sich die erfindungsgemäße Befeuchtungsvorrichtung in besonderem Maße für den netzunabhängigen Betrieb mittels Batterie bzw. Akku.

Gemäß einer besonders bevorzugten Ausführungsform der Erfindung ist der Befeuchtungsbereich räumlich von dem Flüssigkeitsvorratsraum getrennt. Zur bedarfsgerechten Zuleitung des Befeuchtungsmediums aus dem Flüssigkeitsvorratsraum ist vorzugsweise eine Fluidleitungseinrichtung vorgesehen, über welche der Befeuchtungsbereich mit dem Flüssigkeitsvorratsraum in Verbindung steht.

Zwischen dem Befeuchtungsbereich und dem Flüssigkeitsvorratsraum ist gemäß einer bevorzugten Ausführungsform der Erfindung eine Trennwand vorgesehen, die den Befeuchtungsbereich von dem Flüssigkeitsvorratsraum trennt. Die genannte Fluidleitungseinrichtung ist vorzugsweise derart angeordnet, daß diese die Trennwand durchsetzt.

Eine im Hinblick auf eine besonders vorteilhafte Handhabbarkeit und zuverlässige Befüllung des Befeuchtungsbereiches vorteilhafte Ausführungsform der Erfindung ist dadurch gegeben, daß der Flüssigkeitsvorratsraum in Gebrauchsposition der Vorrichtung oberhalb des Befeuchtungsbereiches angeordnet ist. Hierdurch wird es möglich, das Befeuchtungsmedium infolge seiner Schwerkraft in den Befeuchtungsbereich zu leiten. Die Abgabe einer Teilmenge der Flüssigkeit in den Befeuchtungsbereich erfolgt in vorteilhafter Weise in Abhängigkeit von einem Flüssigkeitspegelstand in dem Befeuchtungsbereich. Dadurch wird es möglich, in dem Befeuchtungsbereich permanent eine bestimmte Mindestmenge an Befeuchtungsflüssigkeit zur Verfügung zu halten.

Der Füllstand-in dem Befeuchtungsbereich wird auf vorteilhafte Weise dosiert, indem zur Abgabe einer Teilmenge der Flüssigkeit aus dem Flüssigkeitsvorratsraum der Flüssigkeitsvorratsraum belüftet wird. Die Luft zur Belüftung des Flüssigkeitsvorratsraumes wird hierzu gemäß einer besonders bevorzugten Ausführungsform der Erfindung über den Befeuchtungbereich angesaugt.

Die Steuerung der Luftzufuhr erfolgt vorzugsweise, indem eine Dosier-Leitungseinrichtung vorgesehen ist, die sich zwischen dem Flüssigkeitvorratsraum und dem Befeuchtungsbereich erstreckt, wobei die Leitungseinrichtung eine erste Mündung aufweist, die sich auf Höhe des Flüssigkeitspegels in dem Befeuchtungsbereich befindet, und eine zweite Mündung aufweist, die in den Flüssigkeitsvorratsraum in einen Bereich oberhalb des Vorratsraum-Flüssigkeitspegels in den Vorratsraum mündet. Durch die in dem Befeuchtungsbereich befindliche Flüssigkeit wird hierbei die erste Mündung derart lange bedeckt, bis der Flüssigkeitspegel unter die erste Mündung absinkt. Sobald die erste Mündung freigegeben wird, kann über die Dosierleitungseinrichtung Luft in den Flüssigkeitsraum nachströmen. Infolge der nachströmenden Luft gelangt wieder eine geringe Menge Fluid in den Befeuchtungsbereich und der Pegelstand der Flüssigkeit in dem Befeuchtungsbereich steigt, bis die erste Mündung sich wieder unterhalb des Flüssigkeitsspiegels befindet.

In vorteilhafter Weise ist die Dosier-Leitungseinrichtung durch eine Rohrleitung gebildet, die die Trennwand in vertikaler Richtung durchsetzt.

Die Überleitung der Flüssigkeit aus dem Flüssigkeitsvorratsraum in den Befeuchtungsbereich erfolgt durch einen Rohrzapfen, der sich von der Trennwand aus in einen Bereich unterhalb der ersten Mündung der Dosier-Leitungseinrichtung bzw. der Belüftungsleitungseinrichtung erstreckt.

Eine unter fertigungstechnischen Gesichtspunkten günstig herstellbare sowie robuste Ausführungsform des Befeuchters ist dadurch gegeben, daß die Trennwand und die beiden Fluidleitungseinrichtungen integral ausgebildet sind.

Der Flüssigkeitsvorratsraum ist vorzugsweise durch ein topfartiges Gehäuseteil gebildet. Dieses Gehäuseteil ist vorzugsweise aus einem transparenten oder transluzenten Material gebildet. Durch die Ausbildung des Gehäuseteiles aus einem Kunststoffmaterial wird in vorteilhafter Weise ein Splitterschutz sowie eine nochmalige Verringerung der Wärmeverluste erreicht.

Der Befeuchtungsbereich ist gemäß einer besonders bevorzugten Ausführungsform der Erfindung in einem Wannenelement gebildet. Eine besonders wirkungsvolle Befeuchtung des Atemgases bei weiterhin kompaktem Aufbau kann in vorteilhafter Weise dadurch erreicht werden, daß Luftleitungseinrichtungen vorgesehen sind, die derart angeordnet sind, daß das Wannenelement im wesentlichen quer oder entlang eines Spiralweges durchströmt wird. Hierdurch wird ein intensiver Kontakt des Atemgases mit der in dem Befeuchtungsbereich aufgenommenen Flüssigkeitsteilmenge erreicht.

Eine besonders intensive Befeuchtung des Atemgases kann dadurch erreicht werden, daß eine Heizeinrichtung vorgesehen ist zum Erwärmen der in dem Befeuchtungsbereich bevorrateten Flüssigkeitsteilmenge. Die Heizeinrichtung ist vorzugsweise elektrisch betrieben, beispielsweise durch eine Widerstandsheizung. Die Widerstandsheizung ist vorzugsweise durch ein dünnes folienartiges Element gebildet, das mit einem Bodenbereich des Wannenelementes thermisch gekoppelt ist. Vorzugsweise weist hierzu das Wannenelement einen Bodenabschnitt auf, der aus einem Werkstoff hoher Wärmeleitfähigkeit, insbesondere aus Metall, gebildet ist. Alternativ dazu oder auch in Kombination hiermit, ist es auch möglich, die Heizeinrichtung unmittelbar in einen Wandungsabschnitt, insbesondere Bodenabschnitt, des Befeuchtungsbereiches zu integrieren.

In vorteilhafter Weise ist das die Trennwand bildende Integralteil über eine erste Umfangsdichteinrichtung abdichtend in das Wanneneelement eingesetzt. Das Integralteil umfaßt vorzugsweise auch eine zweite Umfangsdichtungseinrichtung, die in Verbindung mit der Trennwand den Flüssigkeitsvorratsraum abdichtend verschließt. Die derart gebildete Befeuchtungseinheit kann zum Nachfüllen geöffnet werden, indem das Wannenelement von dem den Flüssigkeitsvorratsraum bildenden Gehäuseteil abgenommen wird.

Zur Aufnahme der beschriebenen Befeuchtungseinheit ist gemäß einer besonders bevorzugten Ausführungsform der Erfindung ein Aufstellgehäuse vorgesehen, in welches wenigstens das Wannenelement einsetzbar ist. In vorteilhafter Weise ist das Wannenelement oder das Aufstellgehäuse mit einer Atemschlauchanschlußeinrichtung versehen, zum Anschluß eines Atemschlauches. In dem Bereich der Atemschlauchanschlußeinrichtung ist gemäß einer besonders bevorzugten Ausführungsform der Erfindung eine Zweitschlauchanschlußeinrichtung vorgesehen. Über einen hieran anschließbaren Zweitschlauch von vorzugsweise geringem Durchmesser kann eine Druckmessung in einem der Befeuchtungsvorrichtung abfolgenden Bereich beispielsweise im Bereich eines CO₂-Austauschventiles vorgenommen werden. Die Zweitschlauchanschlußeinrichtung ist vorzugsweise unmittelbar neben einem Atemschlauchanschlußzapfen angeordnet. In vorteilhafter Weise entspricht die seitens der Befeuchtungsvorrichtung vorgesehene Anschlußstruktur für den Atemschlauch und vorzugsweise auch die für den Zweit- insbes. Druckmeßschlauch in ihrem Aufbau der entsprechend an einem CPAP-Gerät vorgesehenen Anschlußstruktur. Dadurch wird auf vorteilhafte Weise eine Kompatibilität der Schlauchanschlüsse sowohl mit dem CPAP-Gerät als auch mit der ggf. zwischengeschalteten Befeuchtungseinrichtung erreicht.

Eine robuste und unter fertigungstechnischen Gesichtspunkten vorteilhafte Ausführungsform ist hierbei dadurch gegeben, daß die Zweitschlauchanschlußeinrichtung und die Atemschlauchanschlußeinrichtung integral mit dem Wannenelement oder dem Aufstellgehäuse ausgebildet sind.

Gemäß einer besonders bevorzugten Ausführungsform der Erfindung weist die Befeuchtungsvorrichtung Anschlussorgane auf die ein unmittelbares Andocken der Befeuchtungseinrichtung an ein entsprechendes CPAP-Gerät ermöglichen.

Das CPAP-Gerät und die Befeuchtungsvorrichtung sind hierzu gemäß einer besonders bevorzugten Ausführungsform der Erfindung derart ausgebildet, daß diese auf sichere Weise koppelbar sind. Hierbei erfolgt vorzugsweise auch eine Koppelung der an der Befeuchtungseinrichtung vorgesehenen Zweitschlauchanschlußeinrichtung mit einer CPAP-geräteseitig vorgesehenen Anschlußeinrichtung.

Unabhängig von den vorangehend beschriebenen Maßnahmen oder auch in vorteilhafter Weise in Kombination hiermit wird die eingangs angegebene, der Erfindung zugrundeliegende Aufgabe auch gelöst durch eine Vorrichtung zur Befeuchtung eines Atemgases mit einem Flüssigkeitsvorratsraum zur Bevorratung einer Flüssigkeit, einem Befeuchtungsbereich zur Befrachtung des Atemgases mit der Flüssigkeit indem das Atemgas in dem Befeuchtungsbereich mit der Flüssigkeit in Kontakt tritt, einer Atemgaszuleitungseinrichtung zur Zuleitung des Atemgases zu dem Befeuchtungsbereich, und- einer Atemgasableitungseinrichtung zur Ableitung des befeuchteten Atemgases aus dem Befeuchtungsbereich wobei der Flüssigkeitsvorratsraum durch ein Gehäuseteil gebildet ist, das mit einem Wannenelement zur Bildung des Befeuchtungsbereiches gekoppelt ist, und daß ein Aufstellgehäuseteil vorgesehen ist, zur Aufnahme einer durch das Gehäuseteil und das Wannenelement gebildeten Einheit

Hinsichtlich eines CPAP-Gerätes wird die eingangs angegebene Aufgabe gelöst durch ein CPAP-Gerät mit einem Außengehäuse, einer in dem Außengehäuse aufgenommen Fördereinrichtung zur Förderung eines Atemgases zu einem Atemgasauslaßanschluß, einer Druckerfassungseinrichtung, einer Steuereinrichtung zur Steuerung der Fördereinrichtung in Abhängigkeit von dem erfaßten Druck und einem Druckmeßanschluß zum Anschluß einer Druckerfassungsleitung wobei der Atemgasauslaßanschluß und der Druckmeßanschluß komplementär zu befeuchterseitig vorgesehenen Anschlußorganen ausgebildet sind

Hierdurch wird auf vorteilhafte Weise ein modulartig aufgebautes CPAP-System geschaffen, das einfach und schnell auch von einem Laien bedarfsgerecht konfiguriert werden kann. Auch in einem vollständig ausgebauten Zustand zeichnet sich das erfindungsgemäße CPAP-Gerätesystem durch eine hohe Komplexität aus und ist überdies als stabile Einheit transportfähig.

Die Erfindung betrifft weiterhin auch eine Atemschlauch-Anschußvorrichtung zur Koppelung eines aus einem flexiblen Material gebildeten Atemschlauches mit einem CPAP-Gerät sowie einen mit einer entsprechenden Anschlußvorrichtung versehenen Atemschlauch.

Derartige Atemschläuche finden insbesondere bei der Therapie schlafbezogener Atmungsstörungen Anwendung. Hierbei wird das Atemgas unter einem vorbestimmten ggf. während eines Atemzyklus alternierenden Überdruck einem Patienten zugeführt, zur Erreichung einer pneumatischen Schienung der oberen Atemwege.

Zur Steuerung des Atemgasdruckes ist es bekannt, über einen Druckmeßschlauch den Druck im Bereich einer Atemmaske oder in einem vorzugsweise hiervon um den etwa 10 bis 15-fachen Innen-Durchmesser des Atemgasschlauches beabstandeten Bereich zu erfassen. Dieser Druckmeßschlauch ist üblicherweise in den Atemschlauch eingeschoben.

Der Atemschlauch kann unmittelbar oder über eine elastische Steckmuffe auf einen seitens eines CPAP-Gerätes vorgesehenen Anschlußzapfen aufgesteckt werden. Der Druckmeßschlauch wird hierbei entweder auf einen koaxial im inneren des Anschlußzapfens vorgesehenen Rohrabschnitt aufgesteckt oder über ein kleines in dem Atemschlauch ausgebildetes Loch aus diesem herausgeführt und separat auf einen entsprechenden am CPAP-Gerät vorgesehenen Druckerfassungs-Anschlußzapfen aufgesteckt. Bei den bekannten Atemschlauch-Anschlußstrukturen mit integrierten Anschlußorganen für einen Druckmeßschlauch besteht das Problem eines vergleichsweise hohen respiratorischen Widerstands sowie einer schwierigen Reinigung. Bei Systemen mit frei heraus geführtem Druckmeßschlauch besteht das Problem, daß der Anschluß des Druckmeßschlauches u.U vergessen wird wodurch es zu einem unzulässig hohen Druckanstieg bei der Atemgaszufuhr kommen kann.

Im Hinblick auf diesen Sachverhalt wird gemäß einem weiteren Lösungsgedanken, ein robustes und einfach handhabbares Atemschlauchsystem geschaffen das sich durch einen vergleichsweise geringen respiratorischen Widerstand auszeichnet und bei welchem auch ohne besondere Aufmerksamkeit eine korrekte Koppelung des Atemschlauches mit einem CPAP-Gerät gewährleistet ist.

Dies wird erfindungsgemäß gelöst durch eine Atemschlauch-Anschlußvorrichtung mit einem Basiskörper, einem in dem Basiskörper gebildeten Atemgasdurchgangskanal, und einem Atemschlauchverbindungsabschnitt zur Aufnahme eines Endabschnittes eines Atemschlauches, die sich dadurch auszeichnet, daß in dem Basiskörper in einem zum Zentrum des Atemgasdurchgangskanal radial versetzten Bereich ein Zusatz-Koppelungsabschnitt ausgebildet ist, zur Koppelung einer Zusatzschlauchleitung mit einer seitens einer Atemgasquelle vorgesehenen komplementären Anschlußstruktur.

Der Basiskörper ist vorzugsweise aus einem elastomeren Material gebildet wodurch eine besonders zuverlässige Abdichtung mit der komplementären Anschlußstruktur sowie eine hinreichende Fixierung des Steckers erreicht werden kann.

Bei der genannten, mit dem Zusatz-Koppelungsabschnitt verbundenen Schlauchleitung handelt es sich im Regelfall um eine Druckmeßleitung. Diese Zusatz-Schlauchleitung kann jedoch auch als Analyseleitung zur Entnahme einer Atemgasprobe oder als Spülleitung zum Austausch verbrauchten Atemgases öder auch als Zufuhrleitung z.B für Sauerstoff ausgebildet sein.

Gemäß einer besonders bevorzugten Ausführungsform der Erfindung entspricht der Durchgangsquerschnitt des Atemgasdurchgangskanales im wesentlichen dem Durchgangsquerschnitt des Atemschlauches. Hierdurch wird auf vorteilhafte Weise vermieden, daß der Anschlußstecker in erheblichem Maße zu einer Erhöhung des respiratorischen Widerstandes beiträgt.

Der Atemgasdurchgangskanal weist vorzugsweise einen im wesentlichen kreisförmigen Querschnitt auf und ist unter leichtem Preß-Sitz auf einen geräteseitig vorgesehenen Anschlußzapfen aufsteckbar. Vorzugsweise ist der Atemgasdurchgangskanal in seinem auf den Anschlußzapfen aufsteckbaren Bereich so ausgebildet, daß die Innenwandung des Anschlußzapfens sich im wesentlichen stufenlos an die Innenwandung des abfolgenden Bereiches des Atemgasdurchgangskanales anschließt.

Der Zusatz-Koppelungsabschnitt ist vorzugsweise durch einen in dem Basiskörper ausgebildeten zylindrischen Bohrungsabschnitt gebildet, der sich im wesentlichen parallel zur Längsmittelachse des Atemgasdurchgangskanales erstreckt. Der Innendurchmesser des Atemgasdurchgangskanales liegt vorzugsweise im Bereich von 15 bis 24mm vorzugsweise 19mm - der Innendurchmesser des Zusatz-Koppelungsabschnittes im Bereich von 3 bis 8mm vorzugsweise bei 4mm.

Eine besonders günstig handhabbare Ausführungsform der Erfindung ist vorzugsweise dadurch gegeben, daß sich im Inneren des Basiskörpers ein Kanalabschnitt erstreckt, der von dem Zusatz-Koppelungsabschnitt in den Atemgasdurchgangskanal führt. Der Kanalabschnitt weist vorzugsweise einen zur Aufnahme der Zusatzschlauchleitung ausreichenden Querschnitt auf.

In vorteilhafter Weise ist die Zusatzschlauchleitung in abdichtender Weise in den Kanalabschnitt eingefügt insbesondere eingeklebt. Die Zusatzschlauchleitung ist vorzugsweise bis zu einer vorderen Stirnseite des Basiskörpers durch den Kanalabschnitt hindurch und in den Koppelungsabschnitt hineingeführt.

In einem der vorderen Stirnseite des Basiskörpers abgewandten Bereich ist in vorteilhafter Weise ein Atemschlauchbefestigungsabschnitt ausgebildet in welchen der Atemschlauch in abdichtender,Weise befestigt insbesondere eingeklebt oder einvulkanisiert ist. Alternativ hierzu oder auch in Kombination mit diesen Maßnahmen ist es auch möglich, in dem Atemschlauchbefestigungsabschnitt eine Innengewindezone auszubilden, die komplementär zur Außenumfangsfläche eines Atmeschlauches der eine Spiraleinlage aufweist, geformt ist.

Auch zum Aternschlauch hin wird gemäß einer bevorzugten Ausführungsform der Erfindung der Innenbereich des Atemgasdurchgangskanales derart ausgebildet, daß ein im wesentlichen stufenloser Übergang in den Atemschlauch erreicht wird. Hierdurch wird ebenfalls eine wirkungsvolle Verringerung des respiratorischen Widerstandes erreicht.

Eine besonders sichere und belastbare Koppelung von Atemschlauch und Steckerstruktur wird dadurch erreicht, daß der Basisabschnitt an den Atemschlauch und/oder die Zusatzschlauchleitung angespritzt ist. Der Basiskörper ist vorzugsweise aus einem insbesondere transparenten oder transluzenten elastomeren Material insbesondere Silikonkautschuk gebildet.

Mit der beschriebenen Anschlußstruktur wird in vorteilhafter Weise ein Atemschlauch für ein CPAP-Gerät geschaffen mit einem Schlauchkörper der aus einem flexiblen Material gebildet ist, einem in dem Schlauchkörper geführten Druckmeßschlauch, und einer am Ende des Schlauchkörpers vorgesehenen Anschluß-Steckerstruktur, wobei die Anschluß-Steckerstruktur aus einem elastomeren Material gebildet ist und in der Anschlußsteckerstruktur ein Kanalabschnitt ausgebildet ist über welchen der Druckmeßschlauch aus einem Atemgasleitungsbereich heraus in einen Koppelungsabschnitt geführt ist der sich seitlich neben einem Atemgasleitungsabschnitt befindet.

Der den Koppelungsabschnitt aufnehmende Bereich der Anschluß-Steckerstruktur steht vorzugsweise nasenartig radial über eine Außenumfangsfläche des Atemschlauchverbindungsabschnitts hervor wodurch eine besonders wirkungsvolle Vorpositionierung des Steckers erreicht werden kann.

Zur Behandlung schlafbezogener Atmungsstörungen ist es bekannt, einem Patienten ein ggf. befeuchtetes Atemgas unter einem vorgegebenen Überdruck zuzuführen. Die Bereitstellung des Atemgases unter Überdruck erfolgt hierbei in den meisten Fällen über drehzahlgeregelte Gebläse. Diese Gebläse sind üblicherweise in einem vorzugsweise schallgedämmten Gehäuseteil aufgenommen und an ein Leitungssystem angeschlossen welches zu einer Befeuchtungseinrichtung oder unmittelbar zu einem Koppelungsabschnitt zum Anschluß eines Atemschlauches führt. Dieser Koppelungsabschnitt ist allgemein als kurzer Rohrzapfen ausgebildet, auf welchen der Atemschlauch in abdichtender Weise aufgesteckt werden kann.

Insbesondere bei CPAP-Geräten zur Bereitstellung vergleichsweise hoher Atemgasdruckpegel hat es sich als vorteilhaft erwiesen den momentanen Druck im Atemschlauch oder innerhalb einer Atemmaske zu erfassen. Hierzu wird üblicherweise ein Druckmeßschlauch verwendet, über welchen der zu überwachende Druck an einer definierten Meßstelle abgegriffen und einem beispielsweise in das CPAP-Gerät integrierten Druckwandler zugeführt wird. Der Druckmeßschlauch wird hierbei ähnlich wie der Atemschlauch auf einen Anschlußzapfen in abdichtender Weise aufgesteckt. Im Hinblick auf die Vielzahl verbreiteter Atemschlauch- Druckmeßschlauch- und Befeuchtersysteme kommt es hier häufig zu Kompatibilitätsproblemen.

Dieses wird gemäß einem weiteren Lösungsgedanken durch ein Anschlußstrukturbauteil für ein CPAP-Gerät mit einer rohrförmigen Atemgasdurchleitungseinrichtung deren Durchgangsquerschnitt im wesentlichen dem Durchgangsquerschnitt eines zum Anschluß daran vorgesehenen Atemschlauches entspricht, und einer Druckmeßschlauchanschlußeinrichtung zum Anschluß eines Druckmeßschlauches, wobei die Atemgasdurchleitungseinrichtung und die Druckmeßschlauchanschlußeinrichtung nebeneinanderliegend angeordnet sind.

Dadurch wird es auf vorteilhafte Weise möglich einen herkömmlichen Atemschlauch, einen herkömmlichen Druckmeßschlauch oder auch einen Atemschlauch mit einem Kombinationsstecker an das entsprechend ausgebildete CPAP-Gerät anzuschließen.

Die Atemgasdurchleitungseinrichtung ist vorzugsweise durch einen Rohrzapfen gebildet dessen Innendurchmesser im wesentlichen dem Innendurchmesser eines Atemschlauches entspricht. Auch die Druckmeßschlauchanschlußeinrichtung ist vorzugsweise durch einen Rohrzapfen gebildet. Ein besonders wirkungsvoller Schutz der beiden Rohrzapfen ist dadurch gegeben, daß die beiden Rohrzapfen in einer Ausnehmung versenkt angeordnet sind.

Eine besonders robuste und unter fertigungstechnischen Gesichtspunkten vorteilhafte Ausführungsform der Erfindung ist dadurch gegeben, daß die Druckmeßschlauchanschlußeinrichtung und die Atemgasdurchleitungseinrichtung integral ausgebildet sind.

Das Anschlußstrukturbauteil ist gemäß einem besonderen Aspekt der vorliegenden Erfindung mit einem Plattenabschnitt versehen, wobei die Atemgasdurchleitungseinrichtung den Plattenabschnitt durchsetzt. Dieser Plattenabschitt bildet vorzugsweise eine Labyrinth-Abdeckung, die mit einem schalldämpfenden Weichmaterial beschichtet ist. Dieses Weichstoffmaterial wirkt in vorteilhafter Weise zugleich als Abdichtung zwischen benachbarten Abschnitten des Labyrinthes.

Eine weitere unter fertigungstechnischen Gesichtspunkten vorteilhafte Ausführungsform der Erfindung ist dadurch gegeben, daß sich beide Rohrzapfen im wesentlichen senkrecht von dem Platenabschnitt aus erstrecken. Das Anschlußstrukturbauteil läßt sich hierbei in besonders vorteilhafter Weise als Kunststoff-Spritzteil mit integral d.h. einstückig an dem Plattenabschnitt angeformten Rohrabschnitten ausbilden.

Der Plattenabschnitt ist in vorteilhafter Weise mit einer Dichtungseinrichtung versehen, zum Aufsetzen des Bauteiles auf einen Labyrinthkasten in abdichtender Weise. Zur Fixierung des Anschlußstrukturbauteiles ist in vorteilhafter Weise eine Steckverbindungseinrichtung vorgesehen, insbesondere zur Fixierung des Strukturbauteiles an einer Bodenstruktur eines CPAP-Gerätes.

Die folgenden Punkte werden diskutiert.
1. Vorrichtung zur Befeuchtung eines Atemgases mit:
   - einem Flüssigkeitsvorratsraum zur Bevorratung einer Flüssigkeit,
   - einem Befeuchtungsbereich zur Befrachtung des Atemgases mit der Flüssigkeit indem das Atemgas in dem Befeuchtungsbereich mit der Flüssigkeit in Kontakt tritt,
   - einer Atemgaszuleitungseinrichtung zur Zuleitung des Atemgases zu dem Befeuchtungsbereich, und
   - einer Atemgasableitungseinrichtung zur Ableitung des befeuchteten Atemgases aus dem Befeuchtungsbereich,
   gekennzeichnet durch eine Teilmengenabgabeeinrichtung zur Weitergabe einer Teilmenge der in dem Flüssigkeitsvorratsraum bevorrateten Flüssigkeit in den Befeuchtungsbereich.
2. Vorrichtung nach Punkt 1, dadurch gekennzeichnet, daß der Befeuchtungsbereich räumlich von dem Flüssigkeitsvorratsraum getrennt ist.
3. Vorrichtung nach Punkt 2, dadurch gekennzeichnet, daß der Befeuchtungsbereich mit dem Flüssigkeitsvorratsraum über eine Fluidleitungseinrichtung in Verbindung steht.
4. Vorrichtung nach wenigstens einem der Punkte 1 bis 3, dadurch gekennzeichnet, daß eine Trennwand vorgesehen ist, die den Befeuchtungsbereich von dem Flüssigkeitsvorratsraum trennt.
5. Vorrichtung nach Punkt 4, dadurch, gekennzeichnet, daß die Fluidleitungseinrichtung die Trennwand durchsetzt.
6. Vorrichtung nach wenigstens einem der Punkte 1 bis 5, dadurch gekennzeichnet, daß der Flüssigkeitsvorratsraum in Gebrauchsposition der Vorrichtung oberhalb des Befeuchtungsbereiches angeordnet ist.
7. Vorrichtung nach wenigstens einem der Punkte 1 bis 6, dadurch gekennzeichnet, daß die Abgabe einer Teilmenge der Flüssigkeit in den Befeuchtungsbereich in Abhängigkeit von einem Flüssigkeitspegel in dem Befeuchtungsbereich veranlaßt wird.
8. Vorrichtung nach wenigstens einem der Punkte 1 bis 7, dadurch gekennzeichnet, daß zur Abgabe einer Teilmenge der Flüssigkeit aus dem Flüssigkeitsvorratsraum der Flüssigkeitsvorratsraum belüftet wird.
9. Vorrichtung nach Punkt 8, dadurch gekennzeichnet, daß die Luft zur Belüftung des Flüssigkeitsvorratsraumes über den Befeuchtungbereich angesaugt wird.
10. Vorrichtung nach Punkt 8 oder 9, dadurch gekennzeichnet, daß eine Dosier-Leitungseinrichtung vorgesehen ist die sich zwischen dem Flüssigkeitvorratsraum und dem Befeuchtungsbereich erstreckt, wobei die Dosier-Leitungseinrichtung eine erste Mündung aufweist die sich auf Höhe des Flüssigkeitspegels in dem Befeuchtungsbereich befindet , und eine zweite Mündung aufweist, die sich in dem Flüssigkeitsvorratsraum in einem Bereich oberhalb des Flüssigkeitspegels in dem Vorratsraum, befindet.
11. Vorrichtung nach Punkt 10, dadurch gekennzeichnet, daß die Dosier-Leitungseinrichtung durch eine Rohrleitung gebildet ist welche die Trennwand in vertikaler Richtung durchsetzt.
12. Vorrichtung nach wenigstens einem der Punkte 1 bis 11, dadurch gekennzeichnet, daß die Fluidleitungseinrichtung durch einen Rohrzapfen gebildet ist, der sich von der Trennwand aus in einen Bereich unterhalb der ersten Mündung der Dosier-Leitungseinrichtung erstreckt.
13. Vorrichtung nach wenigstens einem der Punkte 1 bis 12, dadurch gekennzeichnet, daß die Trennwand und die beiden Leitungseinrichtungen integral ausgebildet sind.
14. Vorrichtung nach wenigstens einem der Punkte 1 bis 13, dadurch gekennzeichnet, daß der Flüssigkeitsvorratsraum durch ein topfartiges Gehäuseteil gebildet ist.
15. Vorrichtung nach wenigstens einem der Punkte 1 bis 14, dadurch gekennzeichnet, daß der Befeuchtungsbereich in einem Wannenelement gebildet ist.
16. Vorrichtung nach Punkt 15, dadurch gekennzeichnet, daß Luftleitungseinrichtungen vorgesehen sind die derart angeordnet sind daß das Wannenelement im wesentlichen quer oder entlang eines Spiralweges durchströmt wird.
17. Vorrichtung nach wenigstens einem der Punkte 1 bis 16, dadurch gekennzeichnet, daß eine Heizeinrichtung vorgesehen ist, zum Erwärmen der in dem Befeuchtungsbereich bevorrateten Flüssigkeitsteilmenge.
18. Vorrichtung nach Punkt 17, dadurch gekennzeichnet, daß die Heizeinrichtung elektrisch betrieben ist.
19. Vorrichtung nach wenigstens einem der Punkte 15 bis 18, dadurch gekennzeichnet, daß das Wannenelement einen Bodenabschnitt aufweist der aus einem Werkstoff hoher Wärmeleitfähigkeit insbesondere aus Metall, gebildet ist.
20. Vorrichtung nach wenigstens einem der Punkte 1 bis 19, dadurch gekennzeichnet, daß das die Trennwand bildende Integralteil über eine erste Umfangsdichteinrichtung abdichtend in das Wanneneelement eingesetzt ist, und daß das Integralteil ferner eine zweite Umfangsdichtungseinrichtung aufweist die in Verbindung mit der Trennwand den Flüssigkeitsvorratsraum abdichtend verschließt.
21. Vorrichtung nach wenigstens einem der Punkte 1 bis 20, dadurch gekennzeichnet, daß ein Aufstellgehäuse vorgesehen ist, zur Aufnahme wenigstens des Wannenelementes.
22. Vorrichtung nach wenigstens einem der Punkte 16 bis 21, dadurch gekennzeichnet, daß das Wannenelement oder das Aufstellgehäuse mit einer Atemschlauchanschlußeinrichtung versehen ist, zum Anschluß eines Atemschlauches.
23. Vorrichtung nach Punkt 22, dadurch gekennzeichnet, daß im Bereich der Atemschlauchanschlußeinrichtung eine Zweitschlauchanschlußeinrichtung vorgesehen ist.
24. Vorrichtung nach Punkt 23, dadurch gekennzeichnet, daß die Zweitschlauchanschlußeinrichtung und die Atemschlauchanschlußeinrichtung integral mit dem Wannenelement oder dem Aufstellgehäuse ausgebildet sind.
25. Vorrichtung zur Befeuchtung eines Atemgases mit einem Flüssigkeitsvorratsraum zur Bevorratung einer Flüssigkeit, einem Befeuchtungsbereich zur Befrachtung des Atemgases mit der Flüssigkeit indem das Atemgas in dem Befeuchtungsbereich mit der Flüssigkeit in Kontakt tritt, einer Atemgaszuleitungseinrichtung zur Zuleitung des Atemgases zu dem Befeuchtungsbereich, und- einer Atemgasableitungseinrichtung zur Ableitung des befeuchteten Atemgases aus dem Befeuchtungsbereich insbesondere nach wenigstens einem der Punkte 16 bis 39, dadurch gekennzeichnet, daß der Flüssigkeitsvorratsraum durch ein Gehäuseteil gebildet ist, das mit einem Wannenelement zur Bildung des Befeuchtungsbereiches gekoppelt ist, und daß ein Aufstellgehäuseteil vorgesehen ist, zur Aufnahme einer durch das Gehäuseteil und das Wannenelement gebildeten Einheit.
26. Vorrichtung nach Punkt 25, dadurch gekennzeichnet, daß das Aufstellgehäuse mit Anschlußeinrichtungen versehen ist, zur Koppelung des Aufstellgehäuses unmittelbar mit einem CPAP-Gerät.
27. CPAP-Gerät mit einem Außengehäuse, einer in dem Außengehäuse aufgenommen Fördereinrichtung zur Förderung eines Atemgases zu einem Atemgasauslaßanschluß, einer Druckerfassungseinrichtung, einer Steuereinrichtung zur Steuerung der Fördereinrichtung in Abhängigkeit von dem erfaßten Druck und einem Druckmeßanschluß zum Anschluß einer Druckerfassungsleitung, dadurch gekennzeichnet, daß der Atemgasauslaßanschluß und der Druckmeßanschluß komplementär zu befeuchterseitig vorgesehenen Anschlußorganen ausgebildet sind.
28. CPAP-Gerät nach Punkt 27, dadurch gekennzeichnet, daß eine ElektroAnschlußeinrichtung vorgesehen ist, zur Schaffung einer elektrischen Verbindung mit einer Befeuchtungsvorrichtung.
29. CPAP-Gerät nach Punkt 28, dadurch gekennzeichnet, daß die Elektroanschlußeinrichtung ein Steckverbindungsorgan aufweist, das bei Ankoppelung der Befeuchtungsvorrichtung in Koppelungsstellung gelangt.
30. CPAP-Gerät nach wenigstens einem der Punkte 27 bis 29, dadurch gekennzeichnet, daß die Koppelungsorgane in einem Seitenbereich insbesondere Stirnseitenbereich des Gerätes angeordnet sind.
31.CPAP-Gerät nach wenigstens einem der Punkte 27 bis 30, dadurch gekennzeichnet, daß die seitens der Befeuchtungsvorrichtung ausgangsseitig vorgesehenen Schlauchanschlußorgane den seitens des CPAP-Gerätes ausgangsseitig vorgesehenen Schlauchanschlußorganen entsprechen.
32.Atemschlauch-Anschlußvorrichtung mit:
   - einem Basiskörper (303),
   - einem in dem Basiskörper (303) gebildeten Atemgasdurchgangskanal (311),
   - einem Atemschlauchverbindungsabschnitt (306) zur Aufnahme eines Endabschnittes eines Atemschlauches (301) derart, daß dieser mit dem Atemgasdurchgangskanal (311) in Verbindung steht,
   - dadurch gekennzeichnet, daß in dem Basiskörper (303) in einem zum Zentrum des Atemgasdurchgangskanales (311) radial versetzten Bereich ein Zusatz-Koppelungsabschnitt (305) ausgebildet ist, zur Koppelung einer Zusatzschlauchleitung (308) mit einer seitens einer Atemgasquelle vorgesehenen komplementären Anschlußstruktur (316,317).
33. Atemschlauch-Anschlußvorrichtung nach Punkt 32, dadurch gekennzeichnet, daß der Basiskörper (303) aus einem elastomeren Material gebildet ist.
34.Atemschlauch-Anschlußvorrichtung nach Punkt 32 oder 33, dadurch gekennzeichnet, daß der Zusatz-Koppelungsabschnitt (305) einen Koppelungsabschnitt für eine durch die Zusatzschlauchleitung gebildete Druckmeßleitung (308) bildet.
35.Atemschlauch-Anschlußvorrichtung nach wenigstens einem der Punkte 32 bis 34, dadurch gekennzeichnet, daß der Durchgangsquerschnitt des Atemgasdurchgangskanales (311) im wesentlichen dem Durchgangsquerschnitt des Atemschlauches (301) entspricht.
36. Atemschlauch-Anschlußvorrichtung nach wenigstens einem der Punkte 32 bis 35, dadurch gekennzeichnet, daß der Atemgasdurchgangskanal (311) einen im wesentlichen kreisförmigen Querschnitt aufweist.
37. Atemschlauch-Anschlußvorrichtung nach wenigstens einem der Punkte 32 bis 36, dadurch gekennzeichnet, daß der Zusatz-Koppelungsabschnitt durch einen in dem Basiskörper (303) ausgebildeten zylindrischen Bohrungsabschnitt gebildet ist, der sich im wesentlichen parallel zur Längsmittelachse des Atemgasdurchgangskanales (311) erstreckt.
38. Atemschlauch-Anschlußvorrichtung nach wenigstens einem der Punkte 32 bis 37, dadurch gekennzeichnet, daß der Innendurchmesser des Atemgasdurchgangskanales (311) im Bereich von 15 bis 24mm vorzugsweise 19mm liegt und daß der Innendurchmesser des Zusatz-Koppelungsabschnittes (305) im Bereich von 3 bis 8mm vorzugsweise 4mm liegt.
39. Atemschlauch-Anschlußvorrichtung nach wenigstens einem der Punkte 32 bis 38, dadurch gekennzeichnet, daß sich im Inneren des Basiskörpers (303) ein Kanalabschnitt (309) erstreckt, der von dem Zusatz-Koppelungsabschnitt (305) in den Atemgasdurchgangskanal (311) führt.
40. Atemschlauch-Anschlußvorrichtung nach Punkt 39, dadurch gekennzeichnet, daß der Kanalabschnitt (309) einen zur Aufnahme der Zusatzschlauchleitung (308) ausreichenden Querschnitt aufweist.
41. Atemschlauch-Anschlußvorrichtung nach wenigstens einem der Punkte 32 bis 40, dadurch gekennzeichnet, daß in den Kanalabschnitt die Zusatzschlauchleitung (308) in abdichtender Weise eingefügt insbesondere eingeklebt oder fixiert ist.
42. Atemschlauch-Anschlußvorrichtung nach wenigstens einem der Punkte 32 bis 41, dadurch gekennzeichnet, daß die Zusatzschlauchleitung (308) bis zu einer vorderen Stirnseite (312) des Basiskörpers (303) durch den Kanalabschnitt (309) hindurch und in den Koppelungsabschnitt (305) hineingeführt ist.
43. Atemschlauch-Anschlußvorrichtung nach wenigstens einem der Punkte 32 bis 42, dadurch gekennzeichnet, daß in einem der vorderen Stirnseite (312) des Basiskörpers (303) abgewandten Bereich ein Atemschlauchbefestigungsabschnitt (306) ausgebildet ist in weichem der Atemschlauch (301) in abdichtender Weise befestigt insbesondere eingeklebt oder einvulkanisiert ist.
44. Atemschlauch-Anschlußvorrichtung nach wenigstens einem der Punkte 32 bis 43, dadurch gekennzeichnet, daß in dem Atemschlauchbefestigungsabschnitt (306) eine Innengewindezone ausgebildet ist, die komplementär zur Außenumfangsfläche eines Atemschlauches (301) der eine Spiraleinlage aufweist, ausgebildet ist.
45. Atemschlauch-Anschlußvorrichtung nach wenigstens einem der Punkte 32 bis 44, dadurch gekennzeichnet, daß der Innenbereich des Atemgasdurchgangskanales (311) derart ausgebildet ist, daß ein im wesentlichen stufenloser Übergang in den Atemschlauch (301) erreicht wird.
46. Atemschlauch-Anschlußvorrichtung nach wenigstens einem der Punkte 32 bis 45, dadurch gekennzeichnet, daß der Basisabschnitt (303) an den Atemschlauch (301) und/oder die Zusatzschlauchleitung (308) angespritzt ist.
47. Atemschlauch-Anschlußvorrichtung nach wenigstens einem der Punkte 32 bis 46, dadurch gekennzeichnet, daß der Basiskörper (303) aus einem insbesondere transparenten oder transluzenten elastomeren Material insbesondere Silikonkautschuk gebildet ist.
48. Atemschlauch für ein CPAP-Gerät mit:
   einem Schlauchkörper (301) der aus einem flexiblen Material gebildet ist,
   einem in dem Schlauchkörper (301) geführten Druckmeßschlauch (308), und
   einer am Ende des Schlauchkörpers (301) vorgesehenen Anschluß-Steckerstruktur (302),
   wobei die Anschluß-Steckerstruktur (302) aus einem elastomeren Material gebildet ist und in der Anschlußsteckerstruktur (302) ein Kanalabschnitt (309) ausgebildet ist über welchen der Druckmeßschlauch (308) aus einem Atemgasleitungsbereich (311) heraus in einen Koppelungsabschnitt (305) geführt ist der sich seitlich neben einem Atemschlauchverbindungsabschnitt (304) befindet.
49. Anschlußstrukturbauteil für ein CPAP-Gerät mit:
   einer rohrförmigen Atemgasdurchleitungseinrichtung deren Durchgangsquerschnitt im wesentlichen dem Durchgangsquerschnitt eines zum Anschluß daran vorgesehenen Atemschlauches entspricht, und
   einer Druckmeßschlauchanschlußeinrichtung zum Anschluß eines Druckmeßschlauches, wobei die Atemgasdurchleitungseinrichtung und die Druckmeßschlauchanschlußeinrichtung nebeneinanderliegend angeordnet sind.
50. Anschlußstrukturbauteil nach Punkt 49, dadurch gekennzeichnet, daß die Atemgasdurchleitungseinrichtung durch einen Rohrzapfen (401) gebildet ist.
51. Anschlußstrukturbauteil nach Punkt 49 oder 50, dadurch gekennzeichnet, daß die Druckmeßschlauchanschlußeinrichtung durch einen Rohrzapfen (402) gebildet ist.
52. Anschlußstrukturbauteil nach einem der Punkte 49 bis 51, dadurch gekennzeichnet, daß die beiden Rohrzapfen (401,402) in einer Ausnehmung (403) versenkt angeordnet sind.
53. Anschlußstrukturbauteil nach wenigstens einem der Punkte 49 bis 52, dadurch gekennzeichnet, daß die Druckmeßschlauchanschlußeinrichtung und die Atemgasdurchleitungseinrichtung integral ausgebildet sind.
54. Anschlußstrukturbauteil nach wenigstens einem der Punkte 49 bis 53, dadurch gekennzeichnet, daß dieses einen Plattenabschnitt aufweist und daß die Atemgasdurchleitungseinrichtung den Plattenabschnitt durchsetzt.
55. Anschlußstrukturbauteil nach Punkt 49, dadurch gekennzeichnet, daß der Plattenabschnitt eine Labyrinth-Abdeckung bildet.
56. Anschlußstrukturbauteil nach Punkt - 54 oder 55, dadurch gekennzeichnet, daß der Plattenabschnitt mit einem schalldämpfenden Weichmaterial beschichtet ist.
57. Anschlußstrukturbauteil nach Punkt 56, dadurch gekennzeichnet, daß das Weichmaterial zwischen benachbarten Abschnitten des Labyrinthes bildet.
58. Anschlußstrukturbauteil nach wenigstens einem der Punkte 49 bis 57, dadurch gekennzeichnet, daß sich die beiden Rohrzapfen im wesentlichen senkrecht von dem Plattenabschnitt aus erstrecken.
59. Anschlußstrukturbauteil nach wenigstens einem der Punkte 49 bis 58, dadurch gekennzeichnet, daß dieses als Kunststoff-Spritzteil ausgebildet ist und die Rohrabschnitte und der Plattenabschnitt integral ausgebildet sind.
60. Anschlußstrukturbauteil nach wenigstens einem der Punkte 49 bis 59, dadurch gekennzeichnet, daß der Plattenabschnitt mit einer Dichtungseinrichtung versehen ist, zum Aufsetzen des Bauteiles auf einen Labyrinthkasten in abdichtender Weise.
61. Anschlußstrukturbauteil nach wenigstens einem der Punkte 49 bis 60, dadurch gekennzeichnet, daß eine Steckverbindungseinrichtung vorgesehen ist, zur Fixierung des Anschlußstrukturbauteiles in einem CPAP-Gerät.
62. Anschlußstrukturbauteil nach wenigstens einem der Punkte 49 bis 61, dadurch gekennzeichnet, daß die Steckverbindungseinrichtung an einer Bodenstruktur eines CPAP-Gerätes fixierbar ist.
63. Vorrichtung zur Zufuhr eines Atemgases unter Überdruck mit:
   - einer Gebläseeinrichtung zur Förderung des Atemgases,
   - einer Gehäuseeinrichtung mit einem Bodenbereich,
   - einem Deckflächenbereich und einem sich zwischen dem Deckflächenbereich und dem Deckelbereich aufwärts erstreckenden Seitenbereich sowie mit einer Anschlußeinrichtung zum Anschluß einer Befeuchtungsvorrichtung zu Befeuchtung des Atemgases, wobei die Anschlußeinrichtung in dem Seitenbereich der Vorrichtung angeordnet ist, derart, daß die Befeuchtungsvorrichtung seitlich ankoppelbar ist.
64. Befeuchtungsvorrichtung für ein CPAP-Gerät mit einem Basisgehäuse und einem Aufnahmebehälter zur Bevorratung von Befeuchtungswasser zum Anschluß an ein CPAP-Gerät, insbesondere nach Punkt 63, dadurch gekennzeichnet, daß die Befeuchtungsvorrichtung eine Anschlußeinrichtung (21) aufweist, die eine Ankoppelung unmittelbar an im Seitenbereich eines CPAP-Gerätes vorgesehene Anschlußorgane (9) ermöglicht, derart, daß die Befeuchtungsvorrichtung (2) sich in Koppelungsstellung neben dem CPAP-Gerät befindet.

Weitere Einzelheiten und Merkmale der Erfindung ergeben sich aus der nachfolgenden Beschreibung eines bevorzugten Ausführungsbeispiels der Erfindung in Verbindung mit der Zeichnung. Es zeigen:
- **Fig. 1**: eine Geräteanordnung, bestehend aus einem CPAP-Gerät und einer unmittelbar an die Front- bzw. Stirnseite seitlich ankoppelbaren Befeuchtungsvorrichtung;
- **Fig. 2**: eine vereinfachte Schnittansicht zur Erläuterung des modularen Aufbaus der CPAP-Geräteanordnung gem. Fig. 1.
- **Fig. 3**: eine vereinfachte Längsschnittansicht durch eine erfindungsgemäße Befeuchtungsvorrichtung;
- **Fig. 4**: eine vereinfachte Schnittansicht entlang der in Fig.3 angegebenen Schnittlinie A-A;
- **Fig. 5**: eine perspektivische Ansicht der Befeuchtungsvorrichtung nach den Figuren 3 und 4 mit Blick auf die zum Anschluß an ein CPAP-Gerät vorgesehenen Steckverbindungsanschlüsse;
- **Fig.6a**: eine perspektivische Ansicht eines CPAP-Gerätes mit einer zur Befeuchtungsvorrichtung komplementären Anschlußstruktur;
- **Fig.6b**: eine perspektivische Ansicht auf die Befeuchtungsvorrichutung nach Fig. 5 jedoch mit Blick auf die atemschlauchseitigen Anschlußstrukturen.

- **Fig. 7**: eine vereinfachte Axialschnittansicht durch einen Endabschnitt eines Atemschlauches und zugehöriger Anschlußvorrichtung;
- **Fig. 8a**: eine Vorderansicht des Basiskörpers der Anschlußvorrichtung:
- **Fig. 8b**: eine Seitenansicht des Atemschlauches mit daran angebrachter Anschlußvorrichtung.
- **Fig. 9**: eine vereinfachte Schnittansicht zur Erläuterung einer geeigneten geräteseitigen komplementären Anschlußstruktur.
- **Fig. 10**: eine perspektivische Ansicht eines Strukturbauteiles gemäß einer bevorzugten Ausführungsform der Erfindung;
- **Fig. 11a,b,c**: drei verschiedene kompatible Anschlußmöglichkeiten.

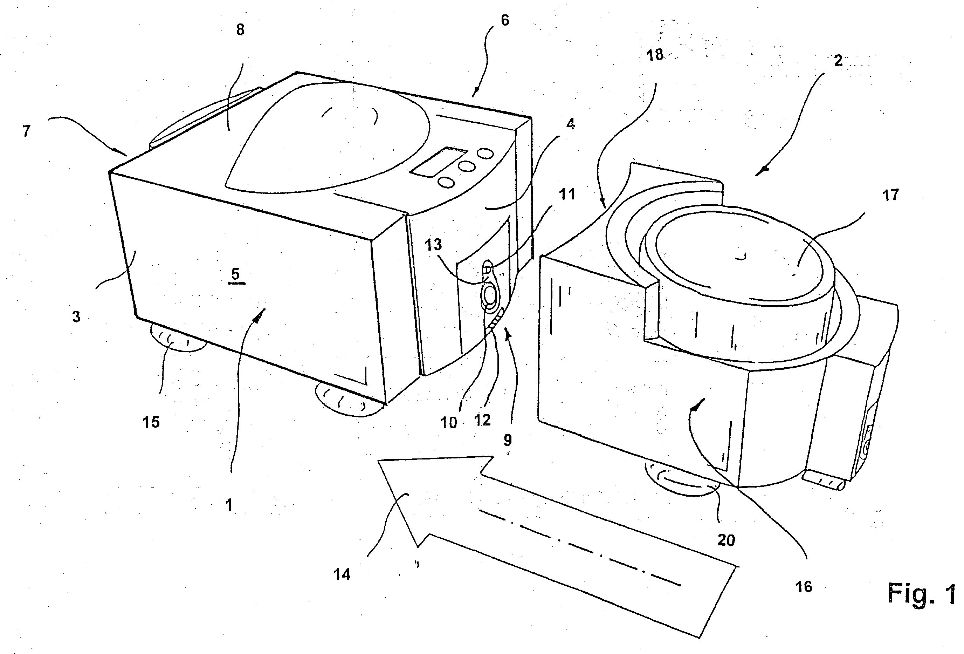

Die in Fig. 1 dargestellte Geräteanordnung umfaßt ein hier allgemein durch das Bezugszeichen 1 gekennzeichnetes CPAP-Gerät und eine daran modular anschließbare Befeuchtungsvorrichtung 2. Das CPAP-Gerät 1 weist hier ein im wesentlichen block- oder kastenförmiges Gehäuse 3 auf, das eine vordere Stirnfläche 4, zwei einander paarweise gegenüberliegende und zueinander im wesentlichen parallele Seitenflächen 5, 6 sowie eine bezogen auf die vordere Stirnfläche 4 im rückwärtigen Bereich des Gehäuses 3 angeordnete Rückseite 7 sowie eine obere Deckfläche 8 aufweist, Im Bereich der vorderen Stirnfläche 4 ist eine Anschlußeinrichtung 9 vorgesehen, die bei der hier dargestellten Ausführungsform einen Atemgasanschlußstutzen 10, einen Druckmeßschlauchanschlußstutzen 11 und eine Elektroanschlußeinrichtung 12 aufweist. Der Atemgasanschlußstutzen 10 und der Druckmeßschlauchanschlußstutzen 11 sind in einer hier nur andeutungsweise dargestellten Ausnehmung 13 im wesentlichen vollständig versenkt angeordnet. Auch die Kontaktelemente einer Elektroanschlußeinrichtung 12 sind in einer Ausnehmung aufgenommen, so daß diese Anschlußorgane ebenfalls nicht oder nicht wesentlich über eine durch die vordere Stirnfläche 4 definierte Fläche überstehen.

Bei der hier dargestellten Ausführungsform ist die vordere Stirnfläche 4 schwach gewölbt ausgebildet, wodurch sich eine besonders wirkungsvolle Unterstützung der Zentrierung der Befeuchtungsvorrichtung 2 ergibt. Der Atemgasanschlußstutzen 10 und der Druckmeßschlauchanschlußstutzen 11 sind derart ausgerichtet, daß dieser sich im wesentlichen parallel zu der durch den Pfeil 14 vereinfacht angedeuteten Fügerichtung erstrecken.

Das CPAP-Gerät 1 weist in seinem Bodenbereich Aufstellorgane (hier Stellfüße 15) auf, die derart ausgebildet sind, daß die Anschlußorgane der Anschlußeinrichtung 9 auf einem vorbestimmten vertikalen Höhenniveau gehalten sind, das exakt auf das entsprechende Höhenniveau der Anschlußorgane der Befeuchtungsvorrichtung 2 abgestimmt ist.

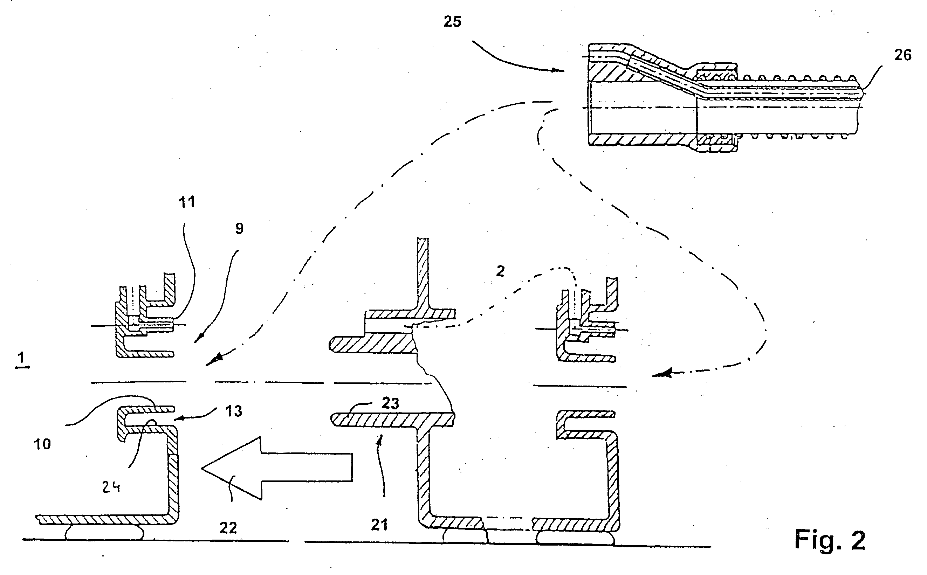

Die Befeuchtungsvorrichtung 2 umfaßt einen Basiskörper 16 und einen hierin aufgenommenen Flüssigkeitsvorratsbehälter 17. Der Flüssigkeitsvorratsbehälter 17 ist beispielsweise zum Nachfüllen von Befeuchtungsflüssigkeit aus dem Basisgehäuse 16 entnehmbar. Das Basisgehäuse weist einen entsprechend komplementär zu der vorderen Stirnfläche 4 des CPAP-Gerätes 1 ausgebildeten Anschlußflächenabschnitt 18 auf, in welchem sich die nachfolgend noch unter Bezugnahme auf Fig. 2 ausführlich erläuterten Anschlußorgane befinden.

Auf einer dem Anschlußflächenabschnitt 18 hier gegenüberliegenden Seite ist das Basisgehäuse 16 wiederum mit Anschlußorganen versehen, die in ihrem Aufbau und in ihrer Anordnung im wesentlichen der bereits bzgl. des CPAP-Gerätes 1 beschriebenen Anschlußeinrichtung 9 entsprechen. Dadurch wird es möglich, die beispielsweise zum Anschluß an das CPAP-Gerät 1 vorgesehenen Schlauchanschlußstecker unmittelbar auch an die Befeuchtungsvorrichtung 2 anzuschließen. Hierbei wird gleichzeitig ein Anschluß des Druckmeßschlauches erreicht.

Die Befeuchtungsvorrichtung 2 weist ebenfalls Stellfüße 20 auf, durch welche die seitens der Befeuchtungsvorrichtung im Bereich des Anschlußflächenabschnittes 18 vorgesehenen Anschlußorgange auf einem vertikalen Höhenniveau gehalten sind, das dem Höhenniveau der Anschlußeinrichtung 9 entspricht.

Wie aus Fig. 2 deutlich hervorgeht, ist die seitens des CPAP-Gerätes 1 vorgesehene Anschlußeinrichtung 9 komplementär zu der seitens der Befeuchtungsvorrichtung 2 vorgesehenen Anschlußeinrichtung 21 ausgebildet. Die beiden Anschlußeinrichtungen 9 und 21 sind, wie durch den Pfeil 22 dargestellt, miteinander in Fügestellung bringbar. Eine besonders wirkungsvolle Vorpositionierung der Anschlußorgane, insbesondere des Atemgasanschlußstutzens 10 und des entsprechenden Gegenstückes 23, wird bei dieser Ausführungsform dadurch erreicht, daß das Gegenstück 23 auch durch die Innenwandung 24 der Ausnehmung 13 zentriert wird. Der Atemgasanschlußstutzen 10 und das seitens der Befeuchtungsvorrichtung 2 vorgesehene Gegenstück 23 befinden sich auf exakt dem gleichen vertikalen Höhenniveau. Ausgangsseitig der Befeuchtungsvorrichtung ist eine Anschlußstruktur vorgesehen, die in ihren wesentlichen Abmessungen der seitens des CPAP-Gerätes vorgesehenen Anschlußstruktur entspricht. Der hier dargestellte Atemschlauchanschlußstecker 25 kann damit bedarfsweise unmittelbar an das CPAP-Gerät 1 oder an die Befeuchtungsvorrichtung 2 angekoppelt werden. Aufgrund einer in die Befeuchtungsvorrichtungs integrierten Druckmeßverbindungsleitung ist auch dann, wenn der Atemschlauchanschlußstecker 25 an die Befeuchtungsvorrichtung 2 angeschlossen ist, eine Verbindung zwischen dem Druckmeßschlauch 26 und dem Druckmeßschlauchanschlußstutzen 11 gegeben. Die vorangehend unter Bezugnahme auf die Fig. 1 und 2 beschriebene. CPAP-Geräteanordnung kann, wie in dem folgenden Anwendungsbespiel beschrieben, verwendet werden.

Zunächst wird davon ausgegangen, daß das CPAP-Gerät 1 bereits auf einer Tischfläche aufgestellt ist, und nunmehr das seitens des CPAP-Gerätes 1 geförderte Atemgas befeuchtet werden soll.

Hierzu wird, wie in Fig. 1 angedeutet, die erfindungsgemäße Befeuchtungsvorrichtung ebenfalls auf die Tischfläche aufgestellt und entlang einer zur Tischfläche parallelen und zur vorderen Stirnfläche des CPAP-Gerätes 1 im wesentlichen senkrechten Fügerichtung auf das CPAP-Gerät aufgesteckt. Hierbei geraten die seitens des CPAP-Gerätes 1 und seitens der Befeuchtungsvorrichtung 2 vorgesehenen Anschlußeinrichtungen 9 und 21 miteinander in Fügestellung. Über eine lediglich in Fig. 1 dargestellte Elektroanschlußeinrichtung 12 erfolgt zudem eine Spannungsversorgung einer seitens der Befeuchtungsvorrichtung 2 vorgesehenen Heizeinrichtung. Sobald die beiden Anschlußeinrichtungen 9, 21 vollständig in Fügestellung gelangt sind, werden die beiden Module in dieser Fügestellung durch eine nicht näher dargestellte Rasteinrichtung fixiert, so daß die Befeuchtungsvorrichtung 2 zuverlässig mit dem CPAP-Gerät gekoppelt ist. Der ursprünglich unmittelbar an das CPAP-Gerät 1 angeschlossene Atemgasschlauch mit integrierter Druckmeßleitung kann über den in Fig. 2 durch das Bezugszeichen 25 gekennzeichneten Atemgasanschlußstecker unmittelbar an die Befeuchtungsvorrichtung angeschlossen werden. Hierdurch ist auch eine entsprechende Koppelung zwischen dem Druckmeßschlauch 16 und dem seitens des CPAP-Gerätes 1 vorgesehenen Druckmeßschlauchanschlußstutzens 11 gegeben.

Zum Einfüllen von Befeuchtungswasser in den Flüssigkeitsvorratsbehälter 17 wird dieser aus dem Basisgehäuse 16 der Befeuchtungsvorrichtung entnommen. Nachdem der Flüssigkeitsvorratsbehälter gefüllt ist, kann dieser wieder in das Basisgehäuse 16 eingesetzt werden. Das aus zwei seitlich ankoppelbaren Modulen mit einer kartuschenartig entnehmbaren Nachfülleinheit gebildete CPAP-Gerätesystem ist nunmehr betriebsbereit.

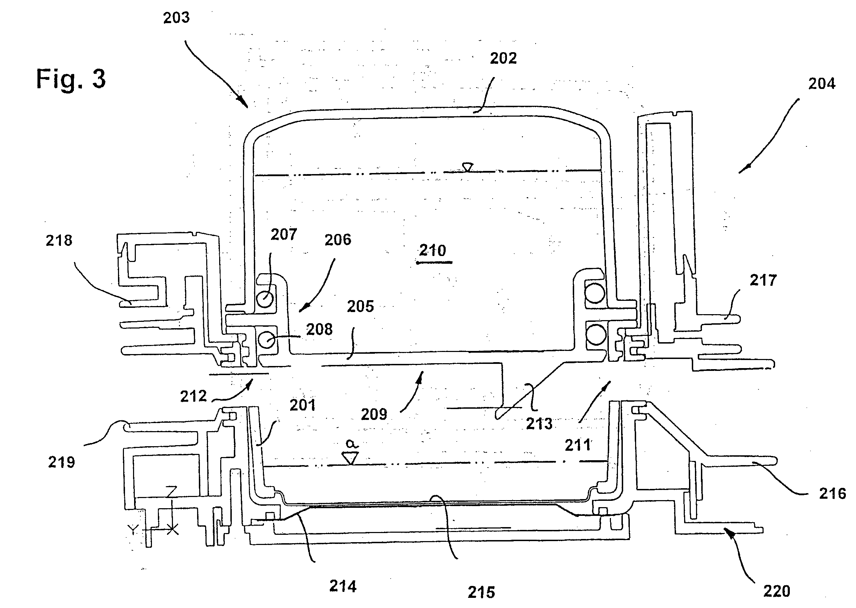

Die Darstellung gem. Fig. 3 zeigt eine Längsschnittansicht durch eine Vorrichtung zur Befeuchtung eines Atemgases (nachfolgend als Befeuchtungsvorrichtung bezeichnet) gem. einer bevorzugten Ausführungsform der Erfindung. Die gezeigte Ausführungsform der Befeuchtungsvorrichtung umfaßt hier eine aus einem Wannenelement 1 und einem damit gekoppelten Topfteil 202 gebildete Nachfülleinheit 203, die auf einfache Weise aus einem hier mehrteilig ausgebildeten Aufstellgehäuse 204 entnommen werden kann.

Das Wannenelement 201 und das Topfteil 202 sind in abdichtender Weise miteinander gekoppelt. Die Koppelung von Wannenelement 201 und Topfteil 202 erfolgt über eine Dichtungsstruktur 206, die bei der hier dargestellten Ausführungsform einen ersten Dichtring 207 und einen zweiten Dichtring 208 aufweist. Die beiden Dichtringe 207 und 208 sind in Umfangsnuten aufgenommen, die in einem Trennelement 209 ausgebildet sind. Das Trennelement 209 weist eine hier integral ausgebildete Trennwand 205 auf.

Die Trennwand 205 trennt den Innenbereich des Topfteils 202 von dem Innenbereich des Wannenelements 201.

In dem Topfteil 202 ist in Verbindung mit der Trennwand 205 ein Flüssigkeitsvorratsraum 10 gebildet, in welchem zunächst der überwiegende Teil der zur Befeuchtung des Atemgases vorgesehenen Flüssigkeit bevorratet ist. In dem unterhalb des Topfteils 202 angeordneten Wannenelement 201 ist ein separater Befeuchtungsbereich gebildet, in dem lediglich eine Teilmenge der Befeuchtungsflüssigkeit aufgenommen ist. Der Pegelstand a, der in dem Wannenelement 201 aufgenommenen Flüssigkeit wird über eine Dosiereinrichtung auf einem vorbestimmten Füllstandsniveau gehalten. Im Zuge des allmählichen Verbrauchs des in dem Wannenelement 201 befindlichen Fluides wird sukzessive oder kontinuierlich aus dem Flüssigkeitsvorratsraum 210 Fluid nachgeführt. Eine bevorzugte Ausführungsform einer hierzu vorgesehenen Dosiereinrichtung wird in Verbindung mit Fig. 2 noch ausführlich beschrieben werden.

Das Wannenelement 201 ist hier im wesentlichen schalenartig ausgebildet und weist eine Atemgaszutrittsöffnung 211 und eine Atemgasaustrittsöffnung 212 auf. Über die Atemgaszutrittsöffnung 211 kann entsprechend der Atemtätigkeit eines Patienten das seitens eines, hier nicht dargestellten, CPAP-Gerätes geförderte Atemgas in das Wannenelement 201 einströmen. Mittels einer hier lediglich vereinfacht dargestellten Umlenkeinrichtung 213 wird das zuströmende Atemgas auf die in dem Wannenelement 201 befindliche Flüssigkeit aufgeleitet. Hierbei reichert sich das zugeführte Atemgas mit Feuchtigkeit an. Das entsprechend befeuchtete Atemgas kann anschließend über die Atemgasaustrittsöffnung 212 abströmen.

Das Wannenelement 201 ist bei der hier dargestellten Ausführungsform mittels einer Heizeinrichtung 214 beheizbar. Die Heizeinrichtung 214 besteht aus einem Heizelement, das in dem Aufstellgehäuse 204 derart angeordnet ist, daß der Bodenbereich des Wannenelements 201 mit dieser in innigen Kontakt treten kann. Zur Steigerung der Wärmeübertragung zwischen den in dem Wannenelement 201 befindlichen Fluid und der Heizeinrichtung 214 ist der Bodenbereich 215 des Wannelements 201 aus einem Werkstoff hoher Wärmeleitfähigkeit, beispielsweise Metall, ausgebildet. Bei der letztgenannten Ausführungsform kann der genannte Bodenbereich 215 beispielsweise im Insert-Molding-Verfahren in den eigentlichen Hauptkörper des Wannenelements 201 eingeformt sein. Das Wannenelement 201 ist derart ausgebildet, daß dieses in leichter Passung in das Aufstellgehäuse 204 selbstpositionierend eingesetzt werden kann. Hierbei fluchten die Atemgaszutrittsöffnung 211 und die Atemgasaustrittsöffnung mit entsprechend komplementär in dem Aufstellgehäuse 204 ausgebildeten Öffnungen bzw. Leitungen.

In dem der Atemgaszutrittsöffnung 211 benachbarten Bereich ist das Aufstellgehäuse 4 mit einem Anschlußstutzen 216 versehen, welcher bei der hier dargestellten Ausführungsform unmittelbar an einen entsprechend komplementär ausgebildeten Anschlußabschnitt eines CPAP-Gerätes aufgesteckt werden kann. In unmittelbarer Nachbarschaft des Anschlußstutzens 216 ist ein weiterer Anschlußstutzen 217 vorgesehen, der mit einem seitens eines CPAP-Gerätes vorgesehenen Druckerfassungsanschluß koppelbar ist. Der Anschlußstutzen 217 bildet Teil eines Leitungssystems, das letztendlich mit dem auf einer gegenüberliegenden Seite der Befeuchtungsvorrichtung vorgesehenen Druckmeßanschlußstutzen 218 in Verbindung steht. An diesen Druckmeßanschluß 218 kann insbesondere ein Druckmeßschlauch angeschlossen werden zum Erfassen des Druckes im Bereich des Atemschlauches, eines Gaswechselventiles oder ggf. auch unmittelbar im Maskenbereich.

Unterhalb des Druckmeßanschlußstutzens 218 ist das Aufstellgehäuse 204 mit einem Atemschlauchanschlußstutzen 219 versehen. Die ausgangsseitig an der Befeuchtungsvorrichtung gebildeten Schlauchanschlußorgane sind derart identisch mit jenem eines CPAP-Gerätes ausgebildet, daß entsprechende Verbindungsschläuche wahlweise entweder direkt an dem CPAP-Gerät angeschlossen werden können oder bedarfsweise bei Verwendung der Befeuchtungsvorrichtung erst an die Ausgangsseite der Befeuchtungsvorrichtung 202. Unterhalb des durch das Bezugszeichen 16 gekennzeichneten Anschlußstutzens ist eine hier nicht dargestellte Steckverbindungseinrichtung vorgesehen, über welche eine elektrische Verbindung zwischen der Heizeinrichtung 214 und einer seitens des CPAP-Gerätes vorgesehenen Spannungsversorgungseinrichtung herstellbar ist. Ggf. ist es auch möglich, über diese Steckverbindungseinrichtung elektrische Signale, beispielsweise Druckmeßsignale zu übertragen.

Das Aufstellgehäuse 204 ist weiterhin mit einer Befestigungseinrichtung 220 versehen, über welche die Befeuchtungsvorrichtung mit einem CPAP-Gerät mechanisch vergleichsweise starr gekoppelt werden kann.

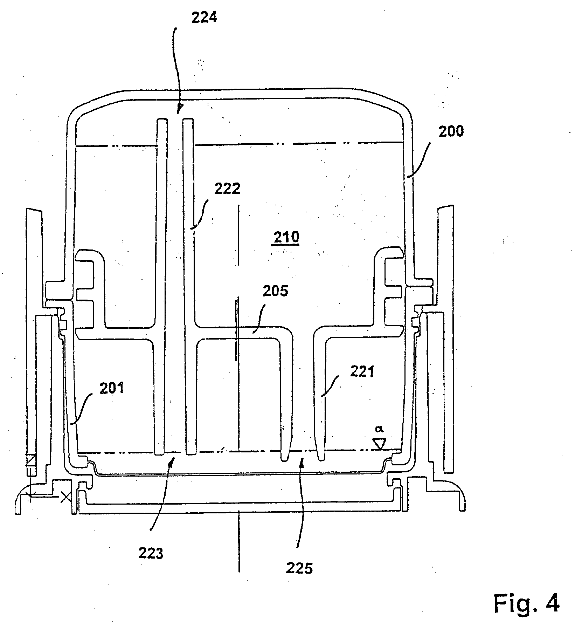

Unter Bezugnahme auf Fig. 4 wird nachfolgend eine bevorzugte Ausführungsform einer Dosiereinrichtung zur Dosierung der in dem Wannenelement 201 befindlichen Fluidmenge beschrieben. Der Flüssigkeitsvorraum 210 und der in dem Wannenelement 201 gebildete Befeuchtungsbereich sind über die Trennwand 205 voneinander getrennt. Über eine Fluidleitungseinrichtung kann bedarfsweise das in dem Flüssigkeitsvorratsraum 210 bevorratete Fluid in den Befeuchtungsbereich übergeleitet werden. Die Steuerung des Fluidnachstromes erfolgt hier durch Steuerung der Luftnachfuhr in den Flüssigkeitsvorratsraum. Bei der hier gezeigten Ausführungsform erfolgt die Regelung der Luftnachfuhr über eine Dosierleitungseinrichtung 222, die ähnlich wie die genannte Fluidleitungseinrichtung 221 die Trennwand 205 vertikal durchsetzt. Die Dosierleitungseinrichtung 222 weist eine erste Mündung 223 und eine zweite Mündung 224 auf. Die erste Mündung 223 ist auf Höhe des Sollpegelstandes a angeordnet. Solange die erste Mündung 223 durch das in dem Wannenelement 201 befindliche Fluid verschlossen ist, kann keine Luft in den Flüssigkeitsvorratsraum 210 nachströmen, so daß wiederum kein Fluid über die Fluidleitungseinrichtung 221 aus dem Flüssigkeitsvorratsraum 210 abfließen kann. Sobald der Pegelstand a unter das Niveau der ersten Mündung abfällt, kann Luft in den Flüssigkeitsvorratsraum nachströmen, wodurch wiederum Fluid aus dem Flüssigkeitsvorratsraum 210 in das Wannenelement 201, bzw. dem hierin gebildeten separaten Befeuchtungsbereich, gelangen kann. Die Fluidleitungseinrichtung 221 weist eine Austrittsmündung 225 auf, die etwas unterhalb des hier durch die Buchstaben a gekennzeichneten Sollpegelstandes liegt.

Die Fluidleitungseinrichtung 221, die Dosierleitungseinrichtung 222 und die Trennwand 5 sind bei der hier gezeigten Ausführungsform durch ein Integralteil gebildet. Zum Einbringen der Flüssigkeit in den Flüssigkeitsvorratsraum ist es möglich, das genannte Integralteil von dem Topfteil 202 abzuziehen. Ggf. kann das Topfteil 202 auch mit einer entsprechenden, abdichtend verschließbaren Nachfüllöffnung versehen sein. Das Topfteil 202, das die Trennwand aufweisende Integralteil und das Wannenelement können jeweils separat gereinigt werden. Die Dosierleitungseinrichtung 222 ist derart ausgebildet, daß die daran vorgesehene zweite Mündung 224 oberhalb des maximalen Füllstandsniveaus des Flüssigkeitsvorratsraumes 210 liegt.

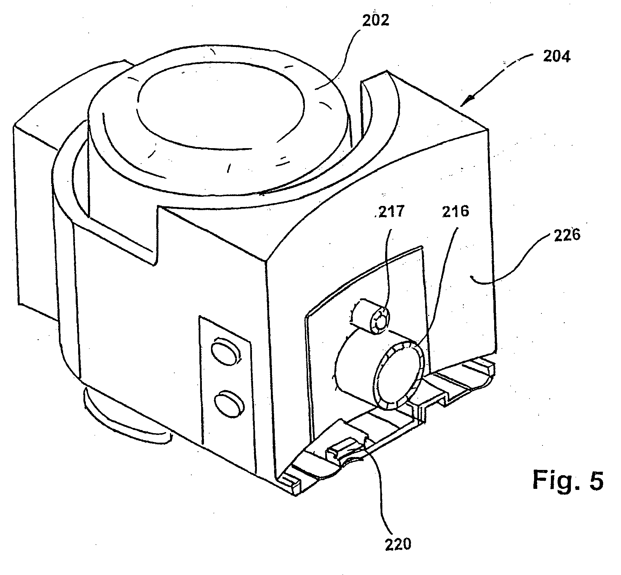

In Fig. 5 ist die vorangehend in Verbindung mit den Fig. 3 und 4 beschriebene Befeuchtungsvorrichtung perspektivisch dargestellt. Das vorzugsweise aus einem transparenten Werkstoff gebildete Topfteil ist hier als im wesentlichen zylindrisch ausgebildeter Becher erkennbar. Dieser Becher ist in einem ebenfalls zylindrischen, in dem Aufstellgehäuse 204 gebildeten Aufnahmeabschnitt eingesetzt. Im Bereich des Topfteils 202 ist das Aufstellgehäuse 204 derart ausgebildet, daß das Topfteil einhändig ergriffen werden kann. Im Bereich der Rückseite 226 der Befeuchtungsvorrichtung sind die bereits in Verbindung mit Fig. 3 beschriebenen Anschlußstutzen 217 bzw. Druckmeßanschlußstutzen 218 vorgesehen. Unterhalb der genannten Anschlußstutzen ist die in Fig. 3 durch das Bezugszeichen 220 gekennzeichnete Befestigungseinrichtung vorgesehen, durch welche eine besonders starre Koppelung der Befeuchtungsvorrichtung mit einem entsprechenden CPAP-Gerät erreicht werden kann. In einer unterhalb des Anschlußstutzens 216 vorgesehenen Aufnahmemulde ist eine hier nicht näher dargestellte Elektro-Steckverbindungseinrichtung vorgesehen zur Schaffung einer elektrischen Verbindung der Heizeinrichtung mit dem zugehörigen CPAP-Gerät.

Im seitlichen Bereich des Außengehäuses sind Schaltorgane 227 vorgesehen, über welche zum einen die Temperatur der Flüssigkeit in dem Wannenelement 201 sowie die Einschaltzeit der Befeuchtungsvorrichtung eingestellt werden können.

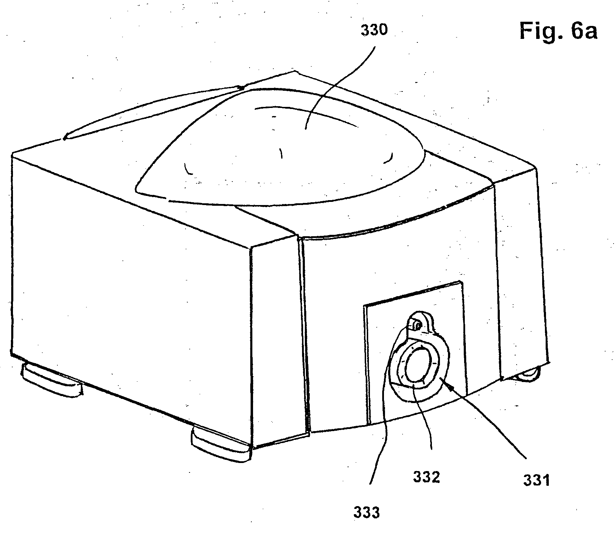

Die Rückseite 226 der Befeuchtungsvorrichtung ist entsprechend der Vorderseite eines nachfolgend in Verbindung mit Fig. 6a beschriebenen CPAP-Gerätes ausgebildet, so daß die Befeuchtungsvorrichtung sich modular nahezu ohne Zwischenraum an das CPAP-Gerät anschließen läßt.

Das in Fig. 6a gezeigte CPAP-Gerät weist ein im wesentlichen quaderförmiges Gehäuse auf in dessen oberen Bereich eine Griffeinrichtung 230 vorgesehen ist über welche das CPAP-Gerät auf ergonomisch vorteilhafte Weise ergriffen werden kann. In einem vorderen Stirnseitenbereich sind Anschlußorgane 231 vorgesehen, zum Anschluß wenigstens eines Atemschlauches.

Bei der gezeigten Ausführungsform sind ein Atemschlauchanschlußzapfen 32 und ein Druckmeßschlauchanschlußzapfen 233 vorgesehen. Die Anordnung dieser Anschlußorgane entspricht im wesentlichen der Anordnung der in Verbindung mit Fig.3 beschriebenen Anschlußorgane 216 und 217. Die Anschlußorgane 231 sind ferner derart ausgebildet, daß die seitens der Befeuchtungseinrichtung (Fig.3) vorgesehenen Anschlußorgane 216, 217 unmittelbar auf- oder eingesteckt werden können. Im Bodenbereich des CPAP-Gerätes sind ferner Eingriffsstrukturen vorgesehen die mit komplementär ausgebildeten Eingriffsabschnitten seitens der Befeuchtungsvorrichtung in Eingriff bringbar sind. Die Anschlußorgane 231 sind hier derart versenkt angeordnet, daß diese nicht über eine Außen- insbesonder Vorderfläche des Gehäuses vorstehen.

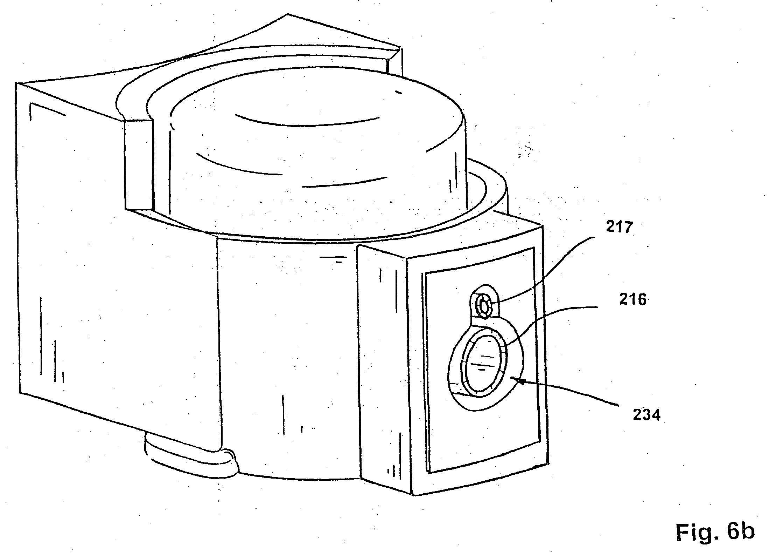

In Fig. 6b ist die vorangehend in Verbindung mit den Figuren 3, 4 und 5 beschriebene Befeuchtungsvorrichtung mit Blick auf deren vorderen Bereich gezeigt. Die Anschlußstutzen 216 und 217 sind ähnlich wie auch seitens des CPAP-Gerätes versenkt angeordnet. Die Anschlußstutzen sind von einem Steckeraufnahmeraum 34 umgeben in welchen eine vorzugsweise aus einem Weichstoffmaterial insbesondere Silikonkautschuk gebildeter Stecker einsteckbar ist.

Der Steckeraufnahmeraum 234 ist vorzugsweise derart ausgebildet, daß ein entsprechender Stecker sowohl auf dem jeweiligen Zapfen 216, 217 als auch entlang der Wandung des Steckeraufnahmeraumes 234 gleitet.

Die Erfindung ist nicht auf die vorangehend beschriebenen Ausführungsbeispiele beschränkt. beispielsweise ist es auch möglich, die beschriebene Befeuchtungsvorrichtung unmittelbar in ein entsprechendes CPAP-Gerät zu integrieren. In das auf einfache Weise an ein CPAP-Gerät andockbare Aufstellgehäuse können auch Nachfülleinheiten eingesetzt werden die in ihrem Aufbau und angewandtem Befeuchtungsprinzip von der beschriebenen Befeuchtungvorrichtung abweichen. Es ist auch möglich, das Wannenelement der Befeuchtungseinheit derart auszubilden, daß dieses unter Verzicht auf das Aufstellgehäuse unmittelbar an das CPAP-Gerät angeschlossen werden kann. Die beschriebene Befeuchtungseinrichtung kann auch unter Zwischenschaltung einer Schlauchleitung mit einer Atemgasquelle verbunden werden. Die Nachfülleinheit kann auch als im wesentlichen wannenartige Einheit unter dem CPAP-Gerät angeordnet werden.

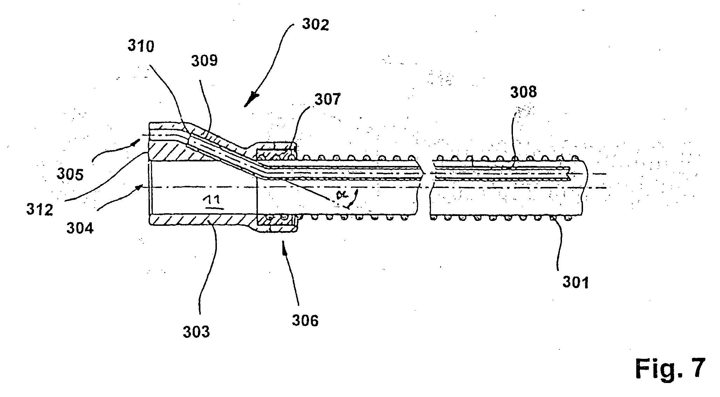

Der in Fig. 7 ist gezeigte Atemschlauch 301 ist in seinem Endbereich mit einer Anschlußvorrichtung 302 versehen die hier einen aus einem elastomeren Material insbes. Silikonkautschuk gebildeten Basiskörper 303 mit zwei eingeformten Koppelungsabschnitten 304,305 aufweist.

Die beiden Koppelungsabschnitte 304, 305 sind integral durch zueinander parallele und im Querschnitt kreisförmige Rohrzonen gebildet. Der Innendurchmesser der jeweiligen Rohrzone ist geringfügig kleiner als der Außendurchmesser der bei angeschlossenem und hierbei leicht geweitetem Stecker in die beiden Rohrzonen eintretenden Anschlußzapfen.

Im schlauchseitigen Bereich des Basiskörpers ist ein Befestigungsabschnitt 306 gebildet in welchem der Atemschlauch 301 über ein Ringelement 307 fixiert ist. Das Ringelement 307 ist hier ebenfalls aus einem elastomeren Material gebildet und mit der Außenfläche des Atemschlauches verklebt.

Im Inneren des Atemschlauches 301 ist ein Zusatzschlauch - hier Druckmeßschlauch 308 geführt. Der Druckmeßschlauch 308 mündet über einen in dem Basiskörper 2 gebildeten Durchführungskanal 309 in den Koppelungsabschnitt 305. Der Druckmeßschlauch 308 ist in den Basiskörper 302 eingeklebt bzw. einvulkanisiert. Der Durchführungskanal 309 ist derart ausgebildet, daß der Druckmeßschlauch 308 nur schwach gekrümmt wird. Der Winkel a zwischen der Längsmittelachse des Koppelungsabschnittes 304 und der Längsmittelachse des Durchführungskanales 309 ist vorzugsweise kleiner als 35°.

Der Übergang der Innenwandung des Druckmeßschlauches 309 in den Koppelungsabschnitt 305 erfolgt hier im wesentlichen stufenlos. hierzu ist ein entsprechender Absatz 310 am Ende des Durchführungskanales 309 ausgebildet.

Auch der in dem Basiskörper 303 gebildete Atemgasleitungsbereich 311 geht hier im wesentlichen stufenlos in den Innenbereich des Atemschlauches 301 über.

Bei entsprechender Elastizität der Schläuche 301, 308 ist es möglich diese bis zur Stirnfläche 312 des Basiskörpers 303 zu führen, so daß die geräteseitigen Koppelungsorgane unmittelbar in die Schläuche 301, 308 eintreten können.

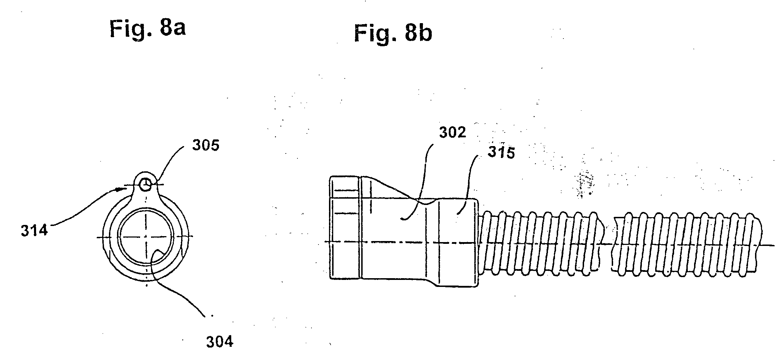

Anhand der Figuren 8a und 8b wird die Außengestalt des Basiskörpers gem. Fig.7 noch deutlicher beschrieben. Wie insbesondere aus Fig.8a deutlich erkennbar ist der zum Anschluß des Zusatzschlauches vorgesehene Koppelungsabschnitt von dem Atemgasleitungs-Koppelungsabschnitt 304 radial beabstandet in einem nasenartig radial auskragenden Bereich 314 des Basiskörpers 303, angeordnet. Hierdurch wird eine wirkungsvolle Vorpositionierung des Basiskörpers in einer geräteseitig vorgesehenen Ausnehmung erreicht.

Dieser nasenartig radial auskragende Bereich fällt zu atemschlauchseitigen Ende des Basiskörpers 303 kontinuierlich ab. Im Bereich des schlauchseitigen Endes ist ein Umfangswulst 315 vorgesehen über welchen ein unter mechanischen Gesichtspunkten günstiger Kraftfluss zwischen Schlauch und Steckerstruktur erreicht wird.

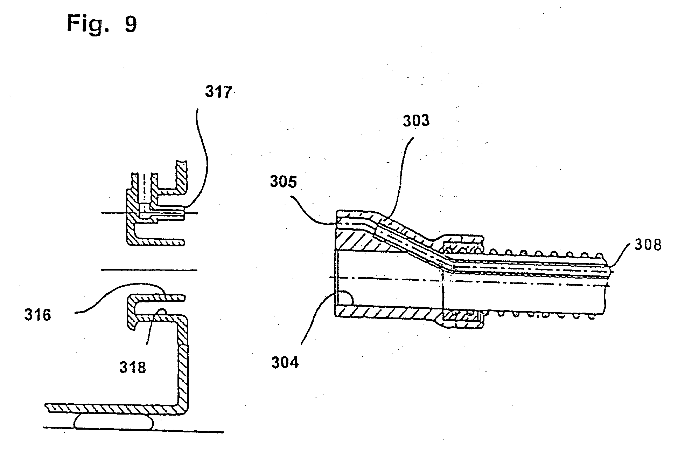

In Fig. 9 ist zum Zwecke der Erläuterung eine bevorzugte Ausführungsform einer geräteseitigen Anschlußstruktur dargestellt die im wesentlichen komplementär zu den in dem Basiskörper 303 des Steckers ausgebildeten Koppelungsabschnitten 304,305 ausgebildet ist.

Der hier durch das Bezugszeichen 316 gekennzeichnete Zapfenabschnitt gelangt in Fügestellung in den Koppelungsabschnitt 304. Der durch das Bezugszeichen 317 gekennzeichnete Zapfenabschnitt gelangt in Fügestellung mit dem Koppelungsabschnitt in Eingriff. Die beiden Zapfenabschnitte 316, 317 sind in einer Ausnehmung 318 versenkt angeordnet. Durch jene die Ausnehmung 318 begrenzende Innenwandung wird in Verbindung mit der in Fig. 8a dargestellten Außenkontur des Basiskörpers 303 eine Vorpositionierung desselben erreicht.

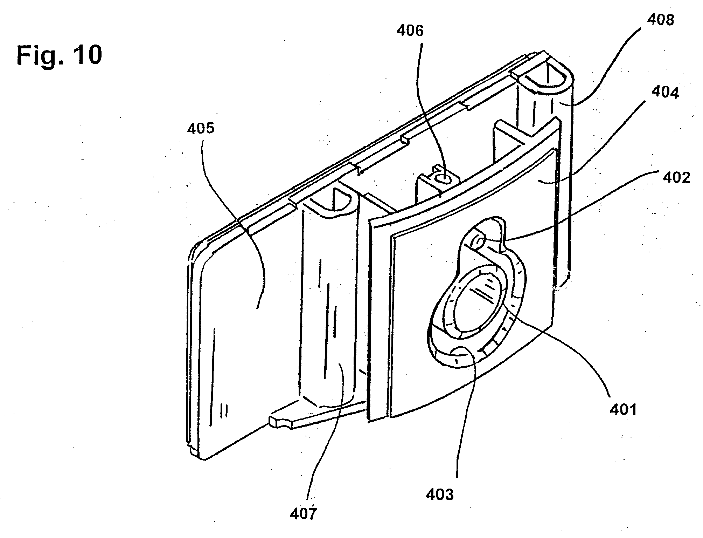

Das in Fig.10 dargestellte Anschlußstrukturbauteil umfaßt eine Atemgasdurchleitungseinrichtung, die hier als Rohrzapfen 401 ausgebildet ist. Diesem Rohrzapfen 401 ist unter Belassung eines Zwischenraumes ein weiterer Rohrzapfen 402 benachbart angeordnet. Dieser Rohrzapfen 402 bildet eine Druckmeßschlauchanschlußeinrichtung. Beide Rohrzapfen 401, 402 sind in einer Ausnehmung 403 versenkt angeordnet. Diese Ausnehmung ist von einer vorderen Abdeckplatte 404 umgeben. Die Abdeckplatte 4 und jene die Ausnehmung 403 begrenzende Wandung sind einstückig ausgebildet.

In einem dem schlauchseitigen Ende des Rohrzapfens 401 abgewandten Bereich mündet dieser in eine Basisplatte 405, die hier eine Abdeckplatte für eine Labyrinthanordnung bildet. Diese hier nicht näher beschriebene Labyrinthanordnung bildet einen verlängerten Atemgasführungsweg zur Absorption etwaiger seitens einer Gebläseeinrichtung erzeugter Geräusche. Die Basisplatte 405 ist auf der hier nicht sichtbaren Rückseite mit einem schallabsorbierenden Material insbes. Schaumstoff beschichtet.

In einem zwischen der Basisplatte 405 und der Abdeckplatte 404 liegenden Bereich ist ein Anschlußkanal 406 ausgebildet über welchen der Innenbereich des Rohrzapfens 402 mit einem auf einer Steuerungsplatine angeordneten Druckwandler koppelbar ist.

Das Anschlußstrukturbauteil ist weiter mit Befestigungseinrichtungen 407, 408 versehen über welche dieses Bauteil in einem CPAP-Gerät auf einfach austauschbare Weise fixierbar ist.

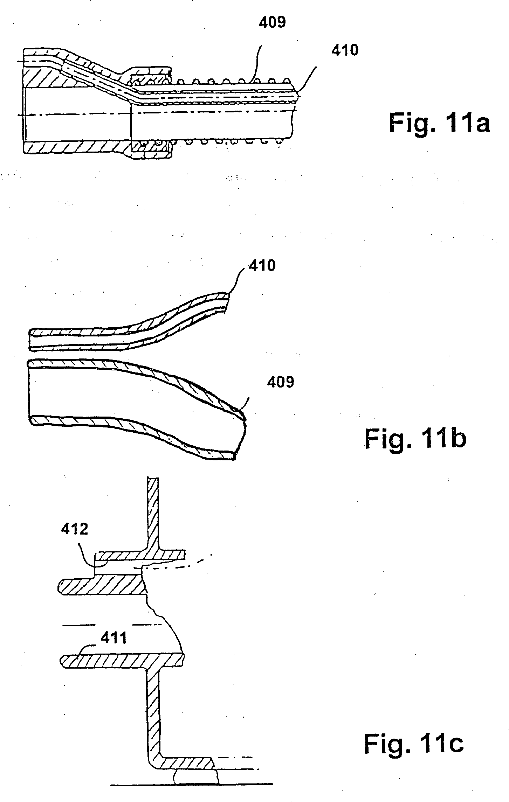

An das gezeigte Anschlußstrukturbauteil sind insbesondere jedoch nicht ausschließlich die in den Figuren 11a, 11b und 11c dargestellten Leitungseinrichtungen anschließbar.

Fig.11a zeigt hierbei einen unter ergonomischen Gesichtspunkten günstig handhabbaren Kompaktstecker mit integrierter Druckmeßschlauchdurchführung.

Fig.11b zeigt einen Atemschlauch 409 und einen hiervon unabhängigen Druckmeßschlauch 410, die beide auch ohne Steckeranordnung unmittelbar über das erfindungsgemäße Anschlußstrukturbauteil an ein entsprechendes CPAP-Gerät angeschlossen werden können.

Fig. 11c zeigt stark vereinfacht einen Koppelungsabschnitt einer Befeuchtungseinrichtung, die unmittelbar über das erfindungsgemäße Strukturbauteil an ein CPAP-Gerät angesetzt werden kann. Hierbei gelangt der durch das Bezugszeichen 411 gekennzeichente Zapfen mit dem Rohrzapfen 401 und der Bohrungsabschnitt 412 mit dem Rohrzapfen 402 in Eingriff.

## Patentansprüche

1. Vorrichtung (1) zur Zufuhr eines Atemgases unter Überdruck mit:
- einer Gebläseeinrichtung zur Förderung des Atemgases,
- einer Gehäuseeinrichtung (3) mit einem Bodenbereich,
- einem Deckflächenbereich (8) und einem sich zwischen dem Deckflächenbereich (8) und dem Bodenbereich aufwärts erstreckenden Seitenbereich sowie mit einer Anschlusseinrichtung (9) zum unmittelbaren Anschluss einer Befeuchtungsvorrichtung (2) zur Befeuchtung des Atemgases, wobei die Anschlusseinrichtung (9) in dem Seitenbereich der Vorrichtung (1) angeordnet ist, derart, dass die Befeuchtungsvorrichtung (2) seitlich so ankoppelbar ist, dass die Vorrichtung (1) und die Befeuchtungsvorrichtung (2) auf einer im Wesentlichen planen Unterlage aufgestellt sind und dass sich die Befeuchtungsvorrichtung (2) in Koppelungsstellung neben der Vorrichtung (1) zur Zufuhr eines Atemgases unter Überdruck befindet.

2. Vorrichtung (1) nach Anspruch 1, **dadurch gekennzeichnet, dass** eine Verrastungseinrichtung vorgesehen ist zum Halten der Befeuchtungsvorrichtung (2) in Fügestellung.

3. Vorrichtung (1) nach Anspruch 2, wobei die Verrastungseinrichtung weiterhin die manuelle Lösbarkeit der Befeuchtungsvorrichtung (2) von der Anschlusseinrichtung (9) ermöglicht.

4. Vorrichtung (1) nach wenigstens einem der vorangegangenen Ansprüche, **dadurch gekennzeichnet, dass** eine Aufstellstruktur (15) vorgesehen ist, die so angeordnet ist, dass die Anschlusseinrichtung (9) auf einem Höhenniveau gehalten sind, das auf das vertikale Höhenniveau der seitens der Befeuchtungsvorrichtung (2) vorgesehenen Anschlussorgane (21) abgestimmt ist.

5. Vorrichtung (1) nach einem der vorangegangenen Ansprüche, **dadurch gekennzeichnet, dass** die Anschlusseinrichtung (9) im Wesentlichen in einer Fügerichtung (14) ausgerichtet ist.

6. Vorrichtung (1) nach wenigstens einem der vorangegangenen Ansprüche, **dadurch gekennzeichnet, dass** die Anschlusseinrichtung (9) Anschlussorgane aufweist und wenigstens eines der Anschlussorgane durch einen Anschlussstutzen (10) gebildet ist.

7. Vorrichtung (1) nach wenigstens einem der vorangegangenen Ansprüche, **dadurch gekennzeichnet, dass** ein Flächenabschnitt der Seite im wesentlichen komplementär zu einem in Fügestellung benachbarten Abschnitt der Befeuchtungsvorrichtung (2) ausgebildet ist.

8. Vorrichtung (1) nach wenigstens einem der vorangegangenen Ansprüche, wobei im Bodenbereich Eingriffsstrukturen vorgesehen sind, die mit komplementär ausgebildeten Eingriffsabschnitten seitens der Befeuchtungsvorrichtung (2) in Eingriff bringbar sind.

9. Vorrichtung (1) nach wenigstens einem der vorangegangenen Ansprüche, **dadurch gekennzeichnet, dass** die Anschlusseinrichtung (9) den besagten Anschlussstutzen (10) zur Durchleitung des Atemgases und einen Leitungsabschnitt (11) zur Ankoppelung einer Druck-Messleitung aufweist.

10. Vorrichtung (1) nach Anspruch 8 mit den Merkmalen von Anspruch 6, **dadurch gekennzeichnet, dass** der Anschlussstutzen (10) und der Leitungsabschnitt (11) zueinander benachbart angeordnet sind.

11. Vorrichtung (1) nach wenigstens einem der vorangegangenen Ansprüche, **dadurch gekennzeichnet, dass** die beiden Anschlussstutzen (10) in einer Ausnehmung (13) angeordnet sind.

12. Vorrichtung (1) nach wenigstens einem der vorangegangenen Ansprüche, **dadurch gekennzeichnet, dass** die genannten Anschlusseinrichtung (9) im Wesentlichen nicht über eine durch die Seitenfläche (4) des Gerätes (1) definierte Flache hervorragt.

13. Vorrichtung (1) nach wenigstens einem der vorangegangenen Ansprüche, **dadurch gekennzeichnet, dass** die Anschlusseinrichtung (9) ferner Elektroanschlussorgane (12) umfasst, zur Schaffung einer elektrischen Verbindung zu der Befeuchtungsvorrichtung (2).

14. Vorrichtung (1) nach Anspruch 13, **dadurch gekennzeichnet, dass** die Elektroanschlussorgane (12) zur Übertragung elektrischer Signale ausgebildet sind, insbesondere zur Spannungsversorgung der Befeuchtungsvorrichtung (2) und/oder zur Übertragung eines Füllstandssignales.

15. Vorrichtung (1) nach wenigstens einem der vorangegangenen Ansprüche, **dadurch gekennzeichnet, dass** Zentrierhilfen vorgesehen sind, wobei vorzugsweise die Innenwandung der den Anschlussstutzen (10) ausnehmenden Ausnehmung auf die Außenumfangsfläche des seitens der Befeuchtungsvorrichtung (2) vorgesehenen Anschlusszapfens (216) abgestimmt ist.

16. Befeuchtungsvorrichtung (2) für eine Vorrichtung (1) zur Zufuhr eines Atemgases unter Überdruck mit einem Basisgehäuse (16) und einem Aufnahmebehälter (17) zur Bevorratung von Befeuchtungswasser zum Anschluss an eine Vorrichtung (1) zur Zufuhr eines Atemgases unter Überdruck, insbesondere nach einem der Ansprüche 1 bis 15, **dadurch gekennzeichnet, dass** die Befeuchtungsvorrichtung (2) eine Anschlusseinrichtung (21) aufweist, die eine Ankoppelung unmittelbar an eine Anschlusseinrichtung (9) ermöglicht, die im Seitenbereich einer Vorrichtung (1) zur Zufuhr eines Atemgases unter Überdruck vorgesehen sind, derart, dass die Befeuchtungsvorrichtung (2) sich in Koppelungsstellung neben der Vorrichtung (1) zur Zufuhr eines Atemgases unter Überdruck befindet, wobei die Befeuchtungsvorrichtung (2) ausgelegt ist, so an die Vorrichtung (1) zur Zufuhr eines Atemgases unter Überdruck gekoppelt zu werden, dass die Vorrichtung (1) zur Zufuhr eines Atemgases unter Überdruck und die Befeuchtungsvorrichtung (2) auf einer im Wesentlichen planen Unterlage aufgestellt sind.

17. Befeuchtungsvorrichtung (2) nach Anspruch 16, **dadurch gekennzeichnet, dass** die Befeuchteranschlusseinrichtung (21) komplementär zu der Anschlusseinrichtung (9) ausgebildet ist, die geräteseitig an der Vorrichtung (1) zur Zufuhr eines Atemgases unter Überdruck vorgesehen ist.

18. Befeuchtungsvorrichtung (2) nach Anspruch 16 oder 17, mit einem Aufstellgehäuse (204) und einer Befestigungseinrichtung (220) zum mechanischen Koppeln der Befeuchtungsvorrichtung (2) mit der Vorrichtung (1) zur Zufuhr eines Atemgases unter Überdruck.

## Claims

1. Apparatus (1) for supplying a respiratory gas under an increased pressure comprising:
- a blowing device for delivering the respiratory gas,
- a housing device (3) having a bottom region,
- a top surface region (8) and a side region extending upwardly between the top surface region (8) and the bottom region, and a connecting device (9) for directly connecting a humidifying apparatus (2) for humidifying the respiratory gas, wherein the connecting device (9) is arranged in the side region of the apparatus (1) such that the humidifying apparatus (2) can be laterally coupled thereto, in such a way that the apparatus (1) and the humidifying apparatus (2) are set up on a substantially flat support and that the humidifying apparatus (2) is disposed in a coupling position beside the apparatus (1) for supplying respiratory gas under an increased pressure.

2. Apparatus (1) as set forth in claim 1 **characterised in that** there is provided a latching device for holding the humidifying apparatus (2) in a joined position.

3. Apparatus (1) as set forth in claim 2 wherein the latching device further facilitates manual releasability of the humidifying apparatus (2) from the connecting device (9).

4. Apparatus (1) as set forth in at least one of the preceding claims **characterised in that** there is provided a support member (15) arranged such that the connecting device (9) is held at a level in respect of height which is matched to the vertical level in respect of height of the connecting members (21) provided on the humidifying apparatus (2).

5. Apparatus (1) as set forth in any one of the preceding claims **characterised in that** the connecting device (9) is oriented substantially in a joining direction (14).

6. Apparatus (1) as set forth in at least one of the preceding claims **characterised in that** the connecting device (9) comprises connecting members and that at least one of the connecting members is formed by a connecting portion (10).

7. Apparatus (1) as set forth in at least one of the preceding claims **characterised in that** a surface portion of the side region is of a substantially complementary configuration to a portion of the humidifying apparatus (2), which portion is adjacent in the joined position.

8. Apparatus (1) as set forth in at least one of the preceding claims wherein in the bottom region there are provided engagement structures which can be brought into engagement with engagement portions of a substantially complementary configuration on the humidifying apparatus (2).

9. Apparatus (1) as set forth in at least one of the preceding claims **characterised in that** the connecting device (9) has said connecting portion (10) for conducting the respiratory gas therethrough and a conduit portion (11) for coupling a pressure-measuring conduit.

10. Apparatus (1) as set forth in claim 8 with the features of claim 6 **characterised in that** the connecting portion (10) and the conduit portion (11) are arranged in mutually adjacent relationship.

11. Apparatus (1) as set forth in at least one of the preceding claims **characterised in that** the two connecting portions (10) are arranged in an opening (13).

12. Apparatus (1) as set forth in at least one of the preceding claims **characterised in that** said connecting device (9) does not project substantially beyond a surface defined by the side region (4) of the apparatus (1).

13. Apparatus (1) as set forth in at least one of the preceding claims **characterised in that** the connecting device (9) further includes electrical connecting members (12) for affording an electrical connection to the humidifying apparatus (2).

14. Apparatus (1) as set forth in claim 13 **characterised in that** the electrical connecting members (12) are adapted for transmitting electrical signals, particularly for supplying voltage to the humidifying apparatus (2) and/or the transmission of a filling level signal.

15. Apparatus (1) as set forth in at least one of the preceding claims **characterised in that** centering aids are provided, wherein preferably the inside wall of the opening which receives the connecting portion (10) is matched to the outside peripheral surface of the connecting projection (216) provided on the humidifying apparatus (2).

16. A humidifying apparatus (2) for an apparatus (1) for supplying a respiratory gas under an increased pressure comprising a base housing (16) and a receiving container (17) for storing humidifying water for connection to an apparatus (1) for supplying a respiratory gas under an increased pressure, in particular as set forth in one of claims 1 through 15, **characterised in that** the humidifying apparatus (2) has a connecting device (21) which permits coupling directly to a connecting device (9) provided in the side region of an apparatus (1) for supplying a respiratory gas under an increased pressure, in such a way that the humidifying apparatus (2) is disposed in the coupling position beside the apparatus (1) for supplying a respiratory gas under an increased pressure, wherein the humidifying apparatus (2) is adapted to be coupled to the apparatus (1) for supplying a respiratory gas under an increased pressure such that the apparatus (1) for supplying a respiratory gas under an increased pressure and the humidifying apparatus (2) are set up on a substantially flat support.

17. A humidifying apparatus (2) as set forth in claim 16 **characterised in that** the humidifier connecting device (21) is of a complementary configuration to the connecting device (9) provided on the side of the apparatus (1) for supplying a respiratory gas under an increased pressure.

18. A humidifying apparatus (2) as set forth in claim 16 or 17 further comprising an installation housing (204) and a fixing device (220) to mechanically couple the humidifying apparatus (2) to the apparatus (1) for supplying a respiratory gas under an increased pressure.

## Revendications

1. Appareil (1) pour le refoulement d'un gaz respirable sous pression positive, comprenant:
- un dispositif de soufflante pour le refoulement du gaz respirable,
- un dispositif de boîtier (3) avec une partie de base,
- une partie de surface supérieure (8) et une partie latérale s'étendant vers le haut entre la partie de surface supérieure (8) et la partie de base, et un dispositif de connexion (9) pour le branchement direct d'un dispositif d'humidification (2) pour l'humidification du gaz respirable, le dispositif de connexion (9) étant disposé dans la partie latérale de l'appareil (1) de telle manière que le dispositif d'humidification (2) peut être accouplé latéralement, permettant ainsi de positionner l'appareil (1) et le dispositif d'humidification (2) sur un support sensiblement plan et de placer le dispositif d'humidification (2) en position d'accouplement à côté de l'appareil (1) pour le refoulement d'un gaz respirable sous pression positive.

2. Appareil (1) selon la revendication 1, **caractérisé en ce qu'**un dispositif de verrouillage est prévu pour le maintien du dispositif d'humidification (2) en position d'assemblage.

3. Appareil (1) selon la revendication 2, où le dispositif de verrouillage permet en outre de débrancher manuellement le dispositif d'humidification (2) du dispositif de connexion (9).

4. Appareil (1) selon au moins une des revendications précédentes, **caractérisé en ce qu'**un élément de support (15) est prévu, monté de telle manière que le dispositif de connexion (9) est maintenu à un niveau de hauteur correspondant au niveau vertical des éléments de connexion (21) prévus sur le dispositif d'humidification (2).

5. Appareil (1) selon une des revendications précédentes, **caractérisé en ce que** le dispositif de connexion (9) est orienté sensiblement suivant une direction d'assemblage (14).

6. Appareil (1) selon au moins une des revendications précédentes, **caractérisé en ce que** le dispositif de connexion (9) comporte des éléments de connexion et **en ce qu'**au moins un des éléments de connexion est formé par une section de connexion (10).

7. Appareil (1) selon au moins une des revendications précédentes, **caractérisé en ce qu'**une zone de surface de la partie latérale présente une configuration sensiblement complémentaire à celle d'une zone du dispositif d'humidification (2) contiguë en position d'assemblage.

8. Appareil (1) selon au moins une des revendications précédentes, où des structures d'enclenchement sont prévues dans la partie de base, lesquelles peuvent être mises en contact avec des parties d'enclenchement configurées de manière complémentaire du dispositif d'humidification (2).

9. Appareil (1) selon au moins une des revendications précédentes, **caractérisé en ce que** le dispositif de connexion (9) comprend la section de connexion (10) pour la conduite du gaz respirable et une section de conduite (11) pour l'accouplement d'une conduite de mesure de pression.

10. Appareil (1) selon la revendication 8 avec les caractéristiques de la revendication 6, **caractérisé en ce que** la section de connexion (10) et la section de conduite (11) sont disposées de manière contiguë l'une à l'autre.

11. Appareil (1) selon au moins une des revendications précédentes, **caractérisé en ce que** les deux sections de connexion (10) sont disposées dans une ouverture (13).

12. Appareil (1) selon au moins une des revendications précédentes, **caractérisé en ce que** le dispositif de connexion (9) ne dépasse sensiblement pas d'une surface définie par la partie latérale (4) de l'appareil (1).

13. Appareil (1) selon au moins une des revendications précédentes, **caractérisé en ce que** le dispositif de connexion (9) comporte en outre des éléments de connexion électrique (12) pour réaliser une liaison électrique au dispositif d'humidification (2).

14. Appareil (1) selon la revendication 13, **caractérisé en ce que** les éléments de connexion électrique (12) sont prévus pour la transmission de signaux électriques, en particulier pour l'alimentation en tension du dispositif d'humidification (2) et/ou pour la transmission d'un signal de niveau de remplissage.

15. Appareil (1) selon au moins une des revendications précédentes, **caractérisé en ce que** des aides au centrage sont prévues, la paroi intérieure de l'ouverture recevant la section de connexion (10) étant préférentiellement ajustée à la surface périphérique extérieure de la broche de connexion (216) prévue sur le dispositif d'humidification (2).

16. Dispositif d'humidification (2) pour un appareil (1) pour le refoulement d'un gaz respirable sous pression positive, avec un boîtier de base (16) et un réservoir (17) pour le stockage de l'eau d'humidification à raccorder à un appareil (1) pour le refoulement d'un gaz respirable sous pression positive, en particulier selon l'une des revendications 1 à 15, **caractérisé en ce que** le dispositif d'humidification (2) comprend un dispositif de connexion (21) permettant un branchement direct à un dispositif de connexion (9) prévu dans la partie latérale d'un appareil (1) pour le refoulement d'un gaz respirable sous pression positive, de telle manière que le dispositif d'humidification (2) se trouve en position d'accouplement à côté de l'appareil (1) pour le refoulement d'un gaz respirable sous pression positive, le dispositif d'humidification (2) étant prévu pour être accouplé à l'appareil (1) pour le refoulement d'un gaz respirable sous pression positive, de telle manière que l'appareil (1) pour le refoulement d'un gaz respirable sous pression positive et le dispositif d'humidification (2) sont positionnés sur un support sensiblement plan.

17. Dispositif d'humidification (2) selon la revendication 16, **caractérisé en ce que** le dispositif de connexion (21) du dispositif d'humidification est configuré de manière complémentaire au dispositif de connexion (9) prévu sur le côté de l'appareil (1) pour le refoulement d'un gaz respirable sous pression positive.

18. Dispositif d'humidification (2) selon la revendication 16 ou la revendication 17, comprenant en outre un boîtier de montage (204) et un dispositif de fixation (220) pour l'accouplement mécanique du dispositif d'humidification (2) à l'appareil (1) pour le refoulement d'un gaz respirable sous pression positive.
